# EUROPEAN PATENT APPLICATION

(11) **EP 3 192 806 A1**
(43) Date of publication of application: **19.07.2017**
(21) Application number: 16020009.3
(22) Date of filing: 13.01.2016
(51) Int. Cl.: C07K 14/735, C12N 15/00, A61M 1/34

(54) **ALPHA CHAIN OF THE HIGH-AFFINITY IGE RECEPTOR (FCERIA)**

(71) Applicant: Affiris AG, 1030 Wien (AT)
(72) Inventor: The designation of the inventor has not yet been filed
(74) Representative: Sonn & Partner Patentanwälte

(57) **Abstract**

Disclosed is an alpha chain of the high-affinity IgE receptor (FceRIa), wherein the amino acid lysine at position 43 (K43) is exchanged with an amino acid selected from the group consisting of alanine, serine, tyrosine, isoleucine, leucine, asparagine, aspartic acid, methionine, phenylalanine, glutamic acid, threonine, glutamine, tryptophan, glycine, and valine, preferably alanine, glycine, serine or tyrosine, especially alanine.

SEQUENCE LISTING

<110>  AFFiRiS AG

<120>  Alpha chain of FceRIa

<130>  R 68475

<160>  20

<170>  PatentIn version 3.5

<210>  1
<211>  257
<212>  PRT
<213>  Homo sapiens

<400>  1

Met Ala Pro Ala Met Glu Ser Pro Thr Leu Leu Cys Val Ala Leu Leu
1               5                   10                  15

Phe Phe Ala Pro Asp Gly Val Leu Ala Val Pro Gln Lys Pro Lys Val
            20                  25                  30

Ser Leu Asn Pro Pro Trp Asn Arg Ile Phe Lys Gly Glu Asn Val Thr
        35                  40                  45

Leu Thr Cys Asn Gly Asn Asn Phe Phe Glu Val Ser Ser Thr Lys Trp
    50                  55                  60

Phe His Asn Gly Ser Leu Ser Glu Glu Thr Asn Ser Ser Leu Asn Ile
65                  70                  75                  80

Val Asn Ala Lys Phe Glu Asp Ser Gly Glu Tyr Lys Cys Gln His Gln
                85                  90                  95

Gln Val Asn Glu Ser Glu Pro Val Tyr Leu Glu Val Phe Ser Asp Trp
            100                 105                 110

Leu Leu Leu Gln Ala Ser Ala Glu Val Val Met Glu Gly Gln Pro Leu
        115                 120                 125

Phe Leu Arg Cys His Gly Trp Arg Asn Trp Asp Val Tyr Lys Val Ile
    130                 135                 140

Tyr Tyr Lys Asp Gly Glu Ala Leu Lys Tyr Trp Tyr Glu Asn His Asn
145                 150                 155                 160

Ile Ser Ile Thr Asn Ala Thr Val Glu Asp Ser Gly Thr Tyr Tyr Cys
                165                 170                 175

Thr Gly Lys Val Trp Gln Leu Asp Tyr Glu Ser Glu Pro Leu Asn Ile

180                    185                    190

Thr Val Ile Lys Ala Pro Arg Glu Lys Tyr Trp Leu Gln Phe Phe Ile
        195                 200                 205

Pro Leu Leu Val Val Ile Leu Phe Ala Val Asp Thr Gly Leu Phe Ile
    210                 215                 220

Ser Thr Gln Gln Gln Val Thr Phe Leu Leu Lys Ile Lys Arg Thr Arg
225                 230                 235                 240

Lys Gly Phe Arg Leu Leu Asn Pro His Pro Lys Pro Asn Pro Lys Asn
                245                 250                 255

Asn

<210>  2
<211>  257
<212>  PRT
<213>  Artificial Sequence

<220>
<223>  Mutant

<220>
<221>  VARIANT
<222>  (43)..(43)
<223>  amino acid selected from A, I, L, N, D, M, F, E, T, Q, W, G and V

<400>  2

Met Ala Pro Ala Met Glu Ser Pro Thr Leu Leu Cys Val Ala Leu Leu
1               5                   10                  15

Phe Phe Ala Pro Asp Gly Val Leu Ala Val Pro Gln Lys Pro Lys Val
            20                  25                  30

Ser Leu Asn Pro Pro Trp Asn Arg Ile Phe Xaa Gly Glu Asn Val Thr
        35                  40                  45

Leu Thr Cys Asn Gly Asn Asn Phe Phe Glu Val Ser Ser Thr Lys Trp
    50                  55                  60

Phe His Asn Gly Ser Leu Ser Glu Glu Thr Asn Ser Ser Leu Asn Ile
65                  70                  75                  80

Val Asn Ala Lys Phe Glu Asp Ser Gly Glu Tyr Lys Cys Gln His Gln
                85                  90                  95

Gln Val Asn Glu Ser Glu Pro Val Tyr Leu Glu Val Phe Ser Asp Trp

            100                 105                 110

Leu Leu Leu Gln Ala Ser Ala Glu Val Val Met Glu Gly Gln Pro Leu
        115                 120                 125

Phe Leu Arg Cys His Gly Trp Arg Asn Trp Asp Val Tyr Lys Val Ile
    130                 135                 140

Tyr Tyr Lys Asp Gly Glu Ala Leu Lys Tyr Trp Tyr Glu Asn His Asn
145                 150                 155                 160

Ile Ser Ile Thr Asn Ala Thr Val Glu Asp Ser Gly Thr Tyr Tyr Cys
                165                 170                 175

Thr Gly Lys Val Trp Gln Leu Asp Tyr Glu Ser Glu Pro Leu Asn Ile
            180                 185                 190

Thr Val Ile Lys Ala Pro Arg Glu Lys Tyr Trp Leu Gln Phe Phe Ile
        195                 200                 205

Pro Leu Leu Val Val Ile Leu Phe Ala Val Asp Thr Gly Leu Phe Ile
    210                 215                 220

Ser Thr Gln Gln Gln Val Thr Phe Leu Leu Lys Ile Lys Arg Thr Arg
225                 230                 235                 240

Lys Gly Phe Arg Leu Leu Asn Pro His Pro Lys Pro Asn Pro Lys Asn
                245                 250                 255

Asn

<210> 3
<211> 257
<212> PRT
<213> Artificial Sequence

<220>
<223> Mutant

<400> 3

Met Ala Pro Ala Met Glu Ser Pro Thr Leu Leu Cys Val Ala Leu Leu
1               5                   10                  15

Phe Phe Ala Pro Asp Gly Val Leu Ala Val Pro Gln Lys Pro Lys Val
            20                  25                  30

Ser Leu Asn Pro Pro Trp Asn Arg Ile Phe Ala Gly Glu Asn Val Thr
        35                  40                  45

Leu Thr Cys Asn Gly Asn Asn Phe Phe Glu Val Ser Ser Thr Lys Trp
    50                  55                  60

Phe His Asn Gly Ser Leu Ser Glu Glu Thr Asn Ser Ser Leu Asn Ile
65                  70                  75                  80

Val Asn Ala Lys Phe Glu Asp Ser Gly Glu Tyr Lys Cys Gln His Gln
                85                  90                  95

Gln Val Asn Glu Ser Glu Pro Val Tyr Leu Glu Val Phe Ser Asp Trp
            100                 105                 110

Leu Leu Leu Gln Ala Ser Ala Glu Val Val Met Glu Gly Gln Pro Leu
        115                 120                 125

Phe Leu Arg Cys His Gly Trp Arg Asn Trp Asp Val Tyr Lys Val Ile
    130                 135                 140

Tyr Tyr Lys Asp Gly Glu Ala Leu Lys Tyr Trp Tyr Glu Asn His Asn
145                 150                 155                 160

Ile Ser Ile Thr Asn Ala Thr Val Glu Asp Ser Gly Thr Tyr Tyr Cys
                165                 170                 175

Thr Gly Lys Val Trp Gln Leu Asp Tyr Glu Ser Glu Pro Leu Asn Ile
            180                 185                 190

Thr Val Ile Lys Ala Pro Arg Glu Lys Tyr Trp Leu Gln Phe Phe Ile
        195                 200                 205

Pro Leu Leu Val Val Ile Leu Phe Ala Val Asp Thr Gly Leu Phe Ile
    210                 215                 220

Ser Thr Gln Gln Gln Val Thr Phe Leu Leu Lys Ile Lys Arg Thr Arg
225                 230                 235                 240

Lys Gly Phe Arg Leu Leu Asn Pro His Pro Lys Pro Asn Pro Lys Asn
                245                 250                 255

Asn

<210>  4
<211>  35
<212>  PRT
<213>  pig

<400>  4

Ala Val Ile Gln Glu Ser Gln Val Ser Leu Asn Pro Pro Trp Asn Arg
1               5                   10                  15

Ile Phe Arg Gly Glu Asn Val Thr Leu Thr Cys Ile Gly Asn Tyr Ser
            20                  25                  30

Leu Glu Asn
        35

<210>  5
<211>  36
<212>  PRT
<213>  Otolemur

<400>  5

Leu Glu Ala Ile Arg Gln Ser Ile Leu Ser Leu Asn Pro Gln Trp Asn
1               5                   10                  15

Arg Ile Phe Lys Gly Glu Asn Val Thr Leu Leu Cys Asn Arg Asp Asn
            20                  25                  30

Phe Phe Glu Val
        35

<210>  6
<211>  49
<212>  PRT
<213>  Mus musculus

<400>  6

Leu Cys Leu Ala Leu Leu Phe Met Ser Leu Asp Val Ile Leu Thr Ala
1               5                   10                  15

Thr Glu Lys Ser Val Leu Thr Leu Asp Pro Pro Trp Ile Arg Ile Phe
            20                  25                  30

Thr Gly Glu Lys Val Thr Leu Ser Cys Tyr Gly Asn Asn His Leu Gln
        35                  40                  45

Met

<210>  7
<211>  49
<212>  PRT
<213>  rattus

<400>  7

Leu Cys Leu Ala Leu Val Leu Ile Ser Leu Gly Val Met Leu Thr Ala
1               5                   10                  15

Thr Gln Lys Ser Val Val Ser Leu Asp Pro Pro Trp Ile Arg Ile Leu
            20                  25                  30

Thr Gly Asp Lys Val Thr Leu Ile Cys Asn Gly Asn Asn Ser Ser Gln
        35                  40                  45

Met

<210>  8
<211>  59
<212>  PRT
<213>  dog

<400>  8

Met Pro Ala Ser Met Gly Gly Pro Ala Leu Leu Trp Leu Ala Leu Leu
1               5                   10                  15

Leu Ser Ser Pro Gly Val Met Ser Ser Asp Thr Leu Lys Pro Thr Val
            20                  25                  30

Ser Met Asn Pro Pro Trp Asn Thr Ile Leu Lys Asp Asp Ser Val Thr
        35                  40                  45

Leu Thr Cys Thr Gly Asn Asn Ser Leu Glu Val
    50                  55

<210>  9
<211>  59
<212>  PRT
<213>  guinea

<400>  9

Met Ala Ala Ala Val Tyr Gly Ser Ala Leu Leu Trp Ile Ala Leu Leu
1               5                   10                  15

Leu Phe Ser Pro Asp Gly Met Ile Met Ala Thr Gln Lys Ser Val Leu
            20                  25                  30

Ser Leu Asn Pro Pro Trp Tyr Arg Val Phe Glu Arg Asp Asn Val Thr
        35                  40                  45

Leu Thr Cys Asn Lys Asn Asn Leu Thr Glu Asp
    50                  55

<210>  10
<211>  59
<212>  PRT
<213>  nomascus

<400>  10

Met Ala Pro Ala Met Glu Ser Pro Thr Leu Leu Cys Val Ala Leu Leu
1               5                   10                  15

Phe Phe Ala Pro Asp Val Val Leu Thr Val Pro Gln Lys Pro Met Val
            20                  25                  30

Ser Leu Asn Pro Pro Trp Asn Arg Ile Phe Lys Gly Glu Asn Val Thr
        35                  40                  45

Leu Thr Cys Asn Gly Asn Asn Phe Phe Glu Val
    50                  55

<210>  11
<211>  59
<212>  PRT
<213>  pongo

<400>  11

Met Ala Pro Ala Met Glu Ser Pro Thr Leu Leu Cys Val Ala Leu Leu
1               5                   10                  15

Phe Phe Ala Pro Asp Gly Val Leu Ala Val Pro Gln Lys Pro Met Val
            20                  25                  30

Ser Leu Asn Pro Pro Trp Asn Arg Ile Phe Lys Gly Glu Asn Val Thr
        35                  40                  45

Phe Thr Cys Asn Gly Asn Asn Phe Phe Glu Val
    50                  55

<210>  12
<211>  59
<212>  PRT
<213>  gorilla

<400>  12

Met Ala Pro Ala Met Glu Ser Pro Thr Leu Leu Cys Val Ala Leu Leu
1               5                   10                  15

Phe Phe Ala Pro Asp Gly Val Leu Ala Val Pro Gln Lys Pro Met Val
            20                  25                  30

Ser Leu Asn Pro Pro Trp Asn Arg Ile Phe Lys Gly Glu Asn Val Thr
        35                  40                  45

Leu Thr Cys Asn Gly Asn Asn Phe Phe Glu Val
    50                  55

<210>  13
<211>  59
<212>  PRT
<213>  masca

<400>  13

Met Ala Pro Ala Met Glu Ser Pro Thr Leu Leu Cys Val Ala Leu Leu
1               5                   10                  15

Phe Phe Ala Pro Asp Gly Val Leu Ala Val Pro Gln Lys Pro Thr Val
            20                  25                  30

Ser Leu Asn Pro Pro Trp Asn Arg Ile Phe Lys Gly Glu Asn Val Thr
        35                  40                  45

Leu Thr Cys Asn Gly Ser Asn Phe Phe Glu Val
    50                  55

<210>  14
<211>  59
<212>  PRT
<213>  saimiri

<400>  14

Met Ala Pro Leu Val Glu Ser Pro Thr Leu Leu Cys Val Ala Leu Leu
1               5                   10                  15

Leu Phe Ala Pro Asp Gly Met Leu Ala Ala Pro Gln Lys Leu Met Ile
            20                  25                  30

Ser Leu Asn Pro Pro Trp Asn Arg Ile Phe Lys Gly Glu Asn Val Thr
        35                  40                  45

Leu Thr Cys Asn Gly Asn Asn Phe Tyr Glu Val
    50                  55

<210>  15
<211>  59
<212>  PRT
<213>  callithrix

<400>  15

Met Ala Pro Leu Met Glu Ser Pro Thr Leu Leu Cys Val Ala Leu Leu
1               5                   10                  15

Leu Phe Ala Pro Asp Gly Val Leu Ala Ala Leu Gln Lys Pro Thr Val
            20                  25                  30

Ser Leu Asn Pro Pro Trp Asn Arg Ile Phe Lys Gly Glu Asn Val Thr
        35                  40                  45

Leu Thr Cys Lys Gly Asn Asn Leu Tyr Glu Val
    50                  55

<210>  16
<211>  59
<212>  PRT
<213>  rabbit

<400>  16

Met Pro Thr Gly Met Gly Gly Pro Ile Leu Leu Cys Ile Ala Phe Leu
1               5                   10                  15

Leu Phe Ser Pro Asp Gly Thr Leu Ala Val Met Leu Lys Ser Val Val
            20                  25                  30

Ser Leu Tyr Pro Pro Trp Asn Arg Ile Phe Arg Gly Glu Ala Val Thr
        35                  40                  45

Leu Thr Cys Asn Gly Asp Lys Ser Leu Asp Asp
    50                  55

<210>  17
<211>  52
<212>  PRT
<213>  myotis

<400>  17

Met Ser Thr Ser Met Gly Gly Pro Ala Leu Leu Trp Val Ala Leu Leu
1               5                   10                  15

Leu Phe Ser Ala Trp Lys Ser Met Met Ser Leu Asn Pro Pro Trp Asn
            20                  25                  30

Arg Ile Phe Arg Gly Glu Asn Val Thr Leu Thr Cys Asn Gly Asn Asn
        35                  40                  45

Ser Leu Asp Val
    50

<210>  18
<211>  60
<212>  PRT
<213>  pteropus

<400>  18

Met Ser Thr Ser Met Gly Gly Pro Ala Leu Leu Trp Ile Ala Leu Leu
1               5                   10                  15

Leu Phe Ser Pro Asp Gly Met Ser Ala Glu Ala Asn Trp Lys Ser Met
            20                  25                  30

Val Ser Leu Asn Pro Pro Trp Asn Arg Ile Phe Arg Gly Glu Asn Val
        35                  40                  45

Thr Leu Thr Cys Asn Gly Asn Lys Ser Pro Glu Val
    50                  55                  60

<210>  19
<211>  55
<212>  PRT
<213>  cow

<400>  19

Met Gly Ala Pro Ala Leu Leu Trp Ile Ala Leu Leu Leu Phe Ser Pro
1               5                   10                  15

Asp Gly Met Ser Ala Ala Ile Trp Lys Ser Lys Val Ser Leu Asn Pro
            20                  25                  30

Pro Trp Arg Lys Ile Leu Lys Gly Asp Ala Val Thr Leu Thr Cys Gly
        35                  40                  45

Thr Asn Gly Ser Ser Glu Asp
    50                  55

<210>  20
<211>  59
<212>  PRT
<213>  orcinus

<400>  20

Met Pro Thr Ala Met Gly Thr Pro Ala Leu Leu Trp Ile Ala Leu Leu
1               5                   10                  15

Leu Phe Ser Pro Asp Gly Ile Ser Ala Ala Ile Trp Arg Ser Lys Val
            20                  25                  30

Ser Leu Asn Pro Pro Trp Asn Arg Ile Phe Arg Gly Glu Asn Val Thr
        35                  40                  45

Leu Thr Cys Gly Gly Asn Asn Ser His Glu Asp
    50                  55

## Description

The present invention relates to improved forms of the alpha chain of the high affinity IgE receptor (FceRI) and methods for use of these improved forms.

### IgE-mediated diseases

In industrialized societies, the prevalence of allergies is currently reaching up to 30% of the population. Allergy can typically progress from mild forms such as allergic rhinoconjunctivitis to severe conditions such as allergic asthma bronchiale with strong restriction of the quality of life. As a consequence, extensive effort has been devoted to developing new therapeutic strategies that target plasma IgE as the key mediator of allergic immune response. With the advent of clinical anti-IgE trials in a variety of (allergic) diseases and co-morbidities, the number of conditions featuring IgE-dependency is increasing (Holgate 2014). More recently, evidence has turned up that IgE also plays a role in extended areas of inflammatory and allergy-related diseases including chronic urticaria, atopic dermatitis, allergic gastroenteropathy and various other (auto-) immune-mediated conditions. Further examples and anecdotic evidence for the causative role of IgE comes from conditions such as e.g. irritable bowel syndrome (Pearson et al. 2015) or idiopathic angioedema (Shroba et al. 2015). As a consequence, the core role of IgE and its downstream signalling has been recognized as paramount targeting opportunity for a broader disease range than initially thought.

As a prototypic example for severe allergic conditions, allergic asthma bronchiale is controlled by a complex interplay between the innate immune system, T-cells, epithelial cell functions and B-cell immunity resulting in production of IgE. Since its recognition as a key mediator of allergy in the 1960ies (Bennich and Ishizaka 1968), IgE has been extensively studied in its clinical and molecular context. The structural and biochemical features of this molecule are well understood and its physiological mode of action via cellular receptors, most notably the high affinity IgE receptor (FceRI) has been well characterized (Miller et al. 1989; Wurzburg et al. 2002; Sutton et al. 2015). IgE production is strongly regulated by IgE itself (Dullaers et al. 2012), suggesting the IgE/FceRI pathway as an attractive targeting opportunity in allergy and IgE-dependent diseases. One special feature of IgE is its very high affinity to the high affinity IgE receptor called FceRI, more specifically to the alpha chain (designated FceRIa unless otherwise specified). The receptor is predominantly expressed on mast cells and basophile granulocytes but also on antigen presenting cells. The tetrameric high affinity IgE receptor is composed of one alpha, one beta and two gamma chains and mediates intracellular signalling upon crosslinking of IgE bound by allergens or immuncomplexes (Galli & Tsai 2012). Crosslinking by allergens thus results in activation of allergy mechanisms notably histamine and cytokine release by mast cell degranulation.

### Anti-IgE therapies and limitations

Since the clinical and market success of the therapeutic anti IgE monoclonal antibody Omalizumab^{®} (Xolair^{®}) (Licari et al. 2015; Incorvaia et al. 2014), there is an increasing demand for affordable and broadly applicable anti-IgE therapeutics. Improved second generation therapeutic antibodies (such as e.g. monoclonal antibodies Ligelizumab^{®}, XmAB7195^{®}, MEDI4212^{®}, Quilizumab^{®} or active anti IgE vaccines) are currently in preclinical and clinical development and evaluation phase.

A major drawback of passive anti-IgE antibody therapy by Omalizumab^{®} are the high cost and relatively high dosing requirements providing that it is necessary to repeatedly inject up to hundreds of milligrams of recombinant antibody into the patient. For several reasons there is a general risk associated with high doses of passively administered, recombinant biologicals such as Omalizumab^{®}: First, the recombinant molecule contains foreign sequences potentially recognized by the patient's immune system. Second, aggregation propensity must always be balanced against the structure and quality of the protein, and finally, Omalizumab^{®} therapy in particular is currently restricted to severe corticoid-resistant asthma patients not exceeding 600 IU/ml plasma IgE. Beside non-responders, the maximum allowed dose for Omalizumab^{®} is 375 mg on a biweekly basis which can pose serious limitations. Other limitations include the risk of anaphylaxia and the need of continuous, well controlled treatment regimes (see Drugs.com http://www.drugs.com/dosage/xolair.html). The cost of goods pose a serious limitation for broader use of Omalizumab^{®} e.g. for less severe but most burdensome manifestations of allergy. The typical treatment protocol for e.g. a 70-80kg patient with 400-500 IU/ml plasma IgE consists of a biweekly anti-IgE dose of 375mg Omalizumab^{®} s.c. Because of dosing and pricing restrictions, the drug can neither be approved for patients with very high IgE levels nor for heavy and overweight patients. Other reasons for restricted use of Omalizumab^{®} include an unfavorable risk to benefit ratio in certain conditions such as food allergy, lack of efficacy or patient compliance or simply the lack of efficacy in a subgroup of asthma patients. Per definition, passively administered anti-IgE antibodies such as Omalizumab^{®} require intrinsically high dosing in order to fulfill efficacy and pharmacodynamic requirements. Half-life and pharmacokinetics of recombinant antibodies such as Omalizumab^{®} are within a restricted window and cannot easily be modulated without extensive developing effort. It is not expected that modifications of Omalizumab^{®} dosing schemes will significantly facilitate dosing restrictions for current anti-IgE therapy or lower the financial burden (Lowe et al. 2015).

In order to circumvent or alleviate the limitations of anti IgE mABs, an alternative combined IgE lowering strategy has been proposed: Extracorporal pre-depletion of high IgE levels followed by passive IgE-lowering anti IgE treatment.

The principle of IgE lowering by apheresis was proposed more than 25 years ago (Sato et al. 1989) who tested the possible use of polyclonal anti IgE antibodies as selective apheresis adsorbers. Major issues in these early studies included not only low efficacy of IgE depletion from plasma but also safety concerns expected from the nature of the adsorber molecule which was a polyclonal goat anti IgE antibody that had the propensity to leak into the blood circulation of the patient. Therefore this non-human adsorber carried the risk of inducing an immune response against foreign epitopes with possible immune complex formation. Similarly, Lebedin and colleagues used monospecific rabbit polyclonal anti-IgE antiserum and a monoclonal antibody or its Fab Fragment as adsorber (Lebedin et al. 1991). In most cases, the major concerns were low plasma depletion efficacy and adsorber material (i.e. non-human antibody material) leaking or shedding into the plasma due to insufficient immobilization of the adsorber to the support matrix or due to proteolytic cleavage.

More recently, Kerzel and colleagues (Kerzel et al. 2011) proposed that specific IgE removal prior to anti IgE therapy would allow Omalizumab^{®} therapy even in very high IgE patients not normally elected for Omalizumab^{®} treatment. The authors applied a clinical setting where plasmapheresis prior to anti IgE therapy could complement conventional anti-IgE therapy especially when Omalizumab^{®} alone was not practicable (Kerzel et al. 2011; Kasperkiewicz et al. 2011). This pilot trial indicated that IgE-specific depletion prior to anti-IgE treatment might be beneficial for high-IgE patients. Zink and colleagues showed that it is possible to target IgE in severe Atopic Dermatitis patients using a combination of apheresis and Omalizumab^{®} (Zink et al. 2012 [Thesis] and Zink et al. 2015).

In this context, a single chain antibody adsorber (designated scFv12) lacking FceRI crosslinking activity was proposed by Lupinek and colleagues (Lupinek et al. 2009) as an alternative to conventional antibodies (Lupinek et al. US 2014/124448 A1). A recent proof-of-concept study entitled "First in Man Trial to Investigate Safety and Efficacy of the New IgE Adsorber" by Fresenius Medical care Deutschland GmbH (Clinical Trial Number NCT02096237; ESPIRA Study; Derfler et al. 2015) has demonstrated that scFv12 can be successfully applied as adsorber for IgE apheresis. A commercial adsorber column designated "IgEnio^{®}"(https://www.fresenius.com/5689 5968.htm) was generated and evaluated on the basis of the monovalent, high affinity scFv12 adsorber. Th authors claimed an IgE reduction by 86.2%, a reduction of Skin Test reactivity and a reduction of allergic symptoms in allergic patients. This particular study was not designed as a combination treatment of IgE apheresis followed by anti IgE treatment. Importantly it could validate the principle of IgE apheresis with the first monovalent IgE adsorber that does not carry the risk of FceRI crosslinking in the case where foreign adsorber protein might leak into blood circulation (such as e.g. common anti IgE antibodies). In parallel to the scFv12 study, a clinical IgE depletion study was launched in 15 atopic dermatitis patients using an adsorber that is based on sheep anti human IgG antibodies (designated Therasorb-IgE by Miltenyi, product ref. # 330-000-571 or 330-000-572; Morren, Clinical Trial NCT02365246) similar to early pilot trials that used polyclonal antibody material from non-human species for IgE adsorption. Again, in these cases, a foreign, non-human adsorber antibody (from sheep), was used providing the risk of inducing anti-sheep IgG-reactive antibodies and some risk of FceRI crosslinking upon shedding of adsorber material from the column into the blood circulation of the patient.

It is well established that non-human proteins such as polyclonal antibodies are not suited for applications as passive vaccine therapeutics because of the induction of serum sickness. In this context it must be stressed that in therapeutic apheresis with selective adsorbers, non-human proteins such as e.g. polyclonal antibodies from animals carry several risks and disadvantages: First, they require careful purification and quality assessment procedures. Second, their affinity is generally lower than affinities of screened and rationally optimized recombinant adsorber proteins. Third, adsorbers must not shed into the blood circulation of the patient during apheresis treatment. Finally, the cost of goods is a decisive criterion for the practicability of apheresis. To date, it is a prerequisite that modern, selective adsorber molecules are produced and purified in sufficient quality at affordable cost. Since immunapheresis or apheresis with selective adsorbers is typically performed in several subsequent apheresis sessions, recyclable or reusable adsorber columns are preferred over expensive single-use adsorber devices.

Some of these features are unfortunately not available from the recently proposed single-chain antibody based adsorber (Lupinek et al. 2009; WO 2012/140214 A1 and "IgEnio^{®}"; Fresenius Medical Care https:// www.fresenius.com /5689 5968 .htm): Importantly, IgEnio^{®} cannot be reused and recycled since it does not efficiently refold after low pH treatment (as demonstrated in EXAMPLE 8 of the example section of the present patent (see below)). Furthermore, bacterially produced, scFv constructs are a priori immunogenic to man since they are recognized as "foreign" because of their non-human structure and origin of sequence material which carries the risk of antibody induction in case of adsorber shedding into the blood stream. In consequence, IgEnio^{®} has been developed as single-use product for apheresis which carries the risk of immunogenicity upon shedding into the blood stream. scFv12 is not intended as a single chain IgE blocker for passive therapeutic use such as e.g. Omalizumab^{®}.

### An alternative to antibody-based IgE binders for extra- or intracorporal application:

As an alternative to mAB- or single chain antibody-based biologicals, it has previously been proposed to use the recombinant, soluble alpha chain of human high affinity IgE receptor (designated FceRIa) as a prototypic highly affine and specific IgE-binding molecule in therapy and diagnostics. As a key feature, FceRIa does not contain any potentially immunogenic (i.e. non-self) sequence material. In nature, FceRI is typically present on basophils and mast cells as a heterotetrameric receptor for IgE. It is a monovalent, high affinity receptor for IgE with complex structural dynamics as extensively described in the literature (for a review, see Sutton et al. 2015). Naturally, the FceRI alpha chain exists as part of the high affinity IgE membrane receptor extending into the extracellular space with two immunoglobulin-like domains that are both essential for correct folding and high affinity binding to IgE (Mallamaci et al. 1993). It is emphasized that both domains of FceRI are required for correct folding of FceRIa and that folding and glycosylation of the protein are the prerequisite for a robust, "natural" candidate IgE adsorber or blocking molecule although artificial measures were proposed e.g. to produce and refold bacterially generated recombinant FceRI or to generate truncated or mutated variants with modified characteristics. More recently, a natural form of shed sFceRI (designated sFceRI) was found in human plasma in free form or complexed with IgE. It was proposed that sFceRI might play a regulatory role for the IgE pathway in that it prevents IgE-binding to surface expressed receptors or that it might contribute to specific ligand blockage similar to e.g. Omalizumab^{®}. However the exact physiological role of natural sFceRI that was shed from membrane anchored FceRI remains to be defined (Platzer et al. 2011).

It is therefore not surprising that FceRIa was soon recognized as candidate IgE blocker or IgE adsorber for therapeutic use. Digan et al. (WO 1998/004718 A1) previously proposed monomeric or dimeric FceRIa/HSA-fusion constructs that could be used as soluble IgE inhibitors for passive anti IgE therapy before the avenue of antibody-based therapies (e.g. Omalizumab^{®}). Digan et al. further proposed FceRI-based *in vitro* diagnostic assays (such as ELISA). One major reason why the development of FceRI-based biologicals was essentially not pursued was its pharmacokinetic behaviour that did not achieve plasma half-life of (humanized) therapeutic antibodies despite artificial measures such as fusing it to serum albumin (WO 1998/004718 A1). Because of its molecular size, plasma half-life of FceRIa is shorter than IgG when passively applied as IgE blocker. Therefore development of modified FceRIa-based constructs with increased size or alternative modifications and formulations might be required to improve plasma half-life. McKenzie et al. (US patent US 5,985,599 A) also proposed the use of FceRIa or fragments thereof as an adsorber for removing immuncomplexes in "plasmapheresis" (i.e. extracorporal IgE depletion) or in a free form as monovalent IgE blocker for preventing IgE signalling.

Huber R et al. previously proposed the use of bacterially generated FceRIa for diagnostic or therapeutic use or for adsorbing IgE (WO 2000/032767 A1). Other prior art publications suggesting FceRI and variants thereof for therapeutic or diagnostic use include e.g. Gould et al. (WO 99/05271 A1) and Hogarth et al. (WO 96/08512 A1, US 8,729,247 B2). Furthermore, other International patent applications such as e.g. Siraganian et al., (WO 89/05352 A1) disclosed a cDNA encoding an IgE receptor alpha-subunit "or its IgE binding fragment" and proposed the use of FceRIa subunits to treat allergies, or to produce entities for *in vitro* diagnostics. Later, Robertson et al. (1993) suggested that a truncated receptor fragment containing only the second, membrane- proximal domain binds to IgE with much lower affinity than a soluble fragment of the receptor containing both domains might be applied therapeutically despite weaker binding to IgE. Furthermore, Yanagihara and colleagues proposed 1994 a recombinant soluble fragment of the human FceRIa receptor that was supposed to down regulate IgE synthesis. More recently, Lingyun Jia et al. (CN 102660569 A) suggested the use a single domain variant of the FceRI alpha chain. As in the McKenzie application, these authors (see CN 102660569 A) mention again the possibility of using one single domain of the FceRIa chain for apheresis. However based on detailed prior art structural and functional studies, it had previously been well established that FceRIa requires two immunoglobulin domains for proper folding and for high affinity binding to IgE (Mallamaci et al. 1993; Garman et al. 1998; Sutton et al. 2015). It is not clear and not demonstrated how these authors could generate a functional, properly folded high affinity FceRIa chain for apheresis. This document is therefore to be regarded as non-enabling with respect to these single domain FceRIa molecules.

Based on this prior art knowledge, it is obvious that the major distinguishing features of FceRIa-based biologicals over antibody-derived IgE binders are (1) the fact that it is a natural, "self"-molecule that will not be recognized by the immune system as a foreign antigenic entity such as e.g. scFv's, nanobodies or other non-natural, AB-like formats with non-human protein sequence material, (2) that its IgE-binding behaviour will strictly depend on structural changes such as e.g. mutations that increase or decrease IgE binding affinity or that affect correct folding and (3) that FceRIa is an excellent, high affinity monovalent IgE binder that lacks crosslinking activity. It is demonstrated in the present invention that along with these advantages, FceRIa shows high robustness in refolding behaviour (EXAMPLE 8 (below)) e.g. as opposed to the single chain adsorber scFv12. It is therefore re-usable when applied for apheresis and thus combines cost effectiveness with the advantage that adsorbed protein material (i.e. IgE) can easily be desorbed by low pH treatment for analytical and quality control purpose. This cannot be realized with adsorbers that are destroyed after desorption by denaturation as demonstrated in EXAMPLE 7 and 8 (see: below, example section). An additional aspect of the FceRIa protein is, that in addition to its main ligand, namely soluble IgE, the FceRIa protein is also bound by anti FceRIa-autoantibodies in plasma from chronic autoimmune urticaria patients (Zubebier et al. 2000) allowing for combined extracorporal depletion of anti-FceRIa autoantibodies and IgE. Taken together, FceRIa combines features that make it an excellent monovalent IgE binder either for extracorporal IgE and/or autoantibody depletion purpose or for providing a soluble anti IgE therapeutic that can be administered similar to biologicals such as e.g. Omalizumab^{®}.

Antibody-related adsorber formats for selective IgE apheresis (such as previously proposed by Sato et al. 1989, Lebedin et al. 1991 or Lupinek et al. 2009) will not provide the features and advantages of an FceRIa-based adsorber as discussed above. On the other hand, parenterally administered therapeutics development with FceRIa-based biologicals for passive IgE targeting was discontinued several years ago, not only because of the success of Omalizumab^{®} but also because of poor serum stability of recombinant FceRIa (even as a serum albumin fusion protein) as previously demonstrated by Digan et al. (WO 1998/004718 A1).

It is an object of the present invention to provide improved means for the prevention and treatment of IgE related diseases.

Therefore, the present invention provides a modified alpha chain of the high-affinity IgE receptor (FceRIa), wherein the amino acid lysine at position 43 (K43) is exchanged with an amino acid selected from the group consisting of alanine, serine, tyrosine, isoleucine, leucine, asparagine, aspartic acid, methionine, phenylalanine, glutamic acid, threonine, glutamine, tryptophan, glycine, and valine, preferably alanine, glycine, serine or tyrosine, especially alanine.

The new FceRIa molecule provided by the present invention has significant advantages over the prior art: It has a significantly higher protease resistance compared to existing forms of FceRIa. On the other side, it does not introduce new immunogenic sites but preserves the adsorption and refolding characteristics of wt FceRI. This makes the molecules according to the present invention advantageous over wt FceRIa, prior art mABs or scFv-based antibody-based adsorbers, especially when used for binding IgE, e.g. in IgE apheresis.

The present molecules are protease resistant and show surprising performance and ability to be re-useable as adsorbed material and devices, they can easily and effectively be regenerated if bound to a column, they can be used as analytics of desorbed material for efficacy clinical monitoring, they allow longer contact of FceRIa with plasma (extra- and intracorporal use of FceRIa), etc..

The present invention therefore provides for the first time a protease resistant form of FceRIa. This is enabled by the present invention by introducing subtle and specific mutations that provide at the same time protease resistance AND low immunogenicity AND no loss of functionality (=IgE binding, pH resistance) because point mutations of FceRIa can functionally modify its binding properties (see e.g. Mackay et al 2002 and similar papers).

This is specifically surprising because the problem of a protease cleavage site at position K43 of FceRIa was not recognised in the prior art. In fact, the data provided with the present invention show that the newly discovered protease cleavage position K43 is the most relevant plasma protease cleavage site when compared to the other predicted or empirically determined cleavage sites (see EXAMPLE 2, below).

Specifically for IgE apheresis, the use of protease labile IgE capturing molecules (such as native FceRIa) is problematic. FceRIa is therefore an IgE capturing molecule which is problematic to be used in apheresis due to the risk that the molecule is degraded by protease activity. Such degradation does not only lead to a decrease in the binding capacity of the apheresis device for IgE, but also represents an important safety issue because these degradation products are potentially contaminating the blood stream of the apheresis patient. With the present invention, a new protease cleavage site of FceRIa was detected (at position 43). Engineering of this protease cleavage site to exclude protease cleavage resulted in a more stable form of FceRIa according to the present invention. As a solution to the problem of providing suitable means for IgE apheresis, the present invention provides an improved variant of FceRIa that combines two essential features: First, cleavage at a newly recognized proteolytic cleavage site within the N-terminal portion of FceRIa must be minimized while at the same time, the structure of FceRIa should *not* be changed in order to preserve IgE binding, pH stability and refolding characteristics as demonstrated in EXAMPLES 5, 6 and 8, below. In order to render FceRIa protease resistant, it is particularly important to avoid the formation of new epitopes that are not normally present within the wild type FceRIa sequence. Besides protease resistance in blood, plasma or serum, the modification of the present invention must guarantee minimal structural changes in order to minimize the risk of de *novo* immunogenic sites. Therefore subtle amino acid changes are preferred over multiple changes or chimaeric FceRIa variants containing larger stretches of non FceRIa sequence At the same time it should preserve the same advantageous desorption and refolding characteristics that wt FceRI can provide. This is demonstrated in EXAMPLES 5, 6 and 8, below.

The basis of the present invention is the unexpected finding that FceRIa is preferentially cleaved by plasma proteases and specifically by plasmin at position K43 (according to UniProt) situated at the N-terminal portion of the molecule.

The amino acid sequence of native human FceRIa is 
1 MAPAMESPTL LCVALLFFAP DGVLAVPQKP KVSLNPPWNR IFKGENVTLT CNGNNFFEVS 60

61 STKWFHNGSL SEETNSSLNI VNAKFEDSGE YKCQHQQVNE SEPVYLEVFS DWLLLQASAE 120

121 VVMEGQPLFL RCHGWRNWDV YKVIYYKDGE ALKYWYENHN ISITNATVED SGTYYCTGKV 180

181 WQLDYESEPL NITVIKAPRE KYWLQFFIPL LVVILFAVDT GLFISTQQQV TFLLKIKRTR 240

214 KGFRLLNPHP KPNPKNN 257
 (SEQ ID No. 1).

The amino acid sequence of the human FceRIa variant according to the present invention is 
1 MAPAMESPTL LCVALLFFAP DGVLAVPQKP KVSLNPPWNR IFXGENVTLT CNGNNFFEVS 60
61 STKWFHNGSL SEETNSSLNI VNAKFEDSGE YKCQHQQVNE SEPVYLEVFS DWLLLQASAE 120
121 VVMEGQPLFL RCHGWRNWDV YKVIYYKDGE ALKYWYENHN ISITNATVED SGTYYCTGKV 180
181 WQLDYESEPL NITVIKAPRE KYWLQFFIPL LVVILFAVDT GLFISTQQQV TFLLKIKRTR 240
214 KGFRLLNPHP KPNPKNN 257
 (SEQ ID No. 2),
wherein "X" at position 43 can be any amino acid selected from A, I, L, N, D, M, F, E, T, Q, W, G and V. Preferred "X" are A, G, S and T; the most promising results have been obtained with "X" being A. This preferred embodiment with respect to human FceRIa therefore has the following amino acid sequence: 
1 MAPAMESPTL LCVALLFFAP DGVLAVPQKP KVSLNPPWNR IFAGENVTLT CNGNNFFEVS 60
61 STKWFHNGSL SEETNSSLNI VNAKFEDSGE YKCQHQQVNE SEPVYLEVFS DWLLLQASAE 120
121 VVMEGQPLFL RCHGWRNWDV YKVIYYKDGE ALKYWYENHN ISITNATVED SGTYYCTGKV 180
181 WQLDYESEPL NITVIKAPRE KYWLQFFIPL LVVILFAVDT GLFISTQQQV TFLLKIKRTR 240
214 KGFRLLNPHP KPNPKNN 257
 (SEQ ID No. 3),

Remarkably, IgE binding is impaired upon cleavage at K43 as demonstrated in EXAMPLE 3. This previously unknown feature seriously limits the usefulness of using recombinant FceRIa for therapeutic purpose in general since IgE binding will be reduced upon contact with the recombinant protein with plasma as demonstrated in EXAMPLE 3. In the course of the present invention, it was recognized that the practical use of FceRIa for extracorporal depletion or intracorporal IgE-specific inhibition or depletion (such as e.g. Omalizumab^{®}) is limited because of the new cleavage mechanism at K43 that was found in the course of the present invention. The responsible protease(s) relevant for the cleavage site provided with the present invention is clearly distinct from postulated (metallo-) proteases that generate shed sFceRI since this previously published "natural" fragment must be cleaved at a membrane-proximal region in order to keep its IgE binding capacity (Platzer et al. 2011). Importantly it was found in the present invention that recombinant FceRIs associates with plasma proteases such as plasmin and thrombin upon contact with human plasma (as demonstrated in EXAMPLE 1) and that cleavage of FceRIa at K43 is caused by plasma proteases, notably plasmin as demonstrated in EXAMPLES 2-5. In consequence, recombinant FceRIa loses its IgE binding capacity whenever it gets into contact with blood, plasma or serum as demonstrated in EXAMPLE 3.

In order to prevent FceRIa from being cleaved at position K43 by plasma proteases, the present invention therefore provides a newly invented, protease-resistant variant of FceRIa that keeps all functional and practical characteristics of natural FceRIa. This new, protease resistant FceRIa variant is specifically beneficial for either extracorporal depletion of IgE or anti FceRI autoantibodies (e.g. by therapeutic apheresis) or for intracorporal use such as e.g. as an IgE inhibitor of soluble IgE similar to Omalizumab®.

Accordingly, the FceRIa according to the present invention has preferably the amino acid sequence according to SEQ ID No. 1 (human FceRIa), wherein the amino acid lysine at position 43 (K43) is exchanged (to become SEQ ID No. 2) with an amino acid selected from the group consisting of alanine (i.e. SEQ ID No. 3), asparagine, aspartic acid, glutamic acid, glutamine, glycine, isoleucine, leucine, methionine, phenylalanine, serine, threonine, tryptophan, tyrosine and valine.

The FceRI alpha chain should preferably contain one most subtle sequence modification that renders the protein entirely protease-insensitive in order to allow repeated or long term use of the adsorber (as demonstrated in EXAMPLE 4). This most preferred variant of FceRIa is illustrated in the example section by the K43→A43 variant (SEQ ID No. 3). However it is also possible but less preferable to use additional or alternative modifications within 3 amino acids N-terminal or 2 amino acids C-terminal of the K43 site. Within the plasmin substrate site consensus surrounding the positively charged K43 cleavage site, it is also possible to substitute F42 alone or in combination with other substitutions by any other amino acid except phenylalanine, tyrosine and tryptophan in order to reduce plasmin sensitivity at K43. In combination or in addition with other substitutions it is also possible to substitute I41 by a charged amino acid or proline or cysteine which will also reduce plasmin sensitivity at K43. In combination or in addition with other substitutions it is also possible to change R40 to any other amino acid except arginine, threonine, valine, glycine, lysine, phenylalanine and isoleucine in order to reduce plasmin sensitivity at K43 based on previous plasmin substrate consensus site studies such as e.g. by Backes at al. 2000 and Hervio et al. 2000. In combination or in addition with the above mentioned substitutions, it is also possible but less preferable to change G44 to any other amino acid except glycine, arginine, lysine or serine in order to reduce plasmin substrate recognition. According to the evolutionary conservation of this region (see zoo-alignment above) and the plasmin substrate site consensus as suggested by Backes at al. 2000, Yuan et al. 2009 or Hervio et al. 2000 it is for example possible to reduce proteolytic cleavage by changing one or several neighbouring amino acids flanking K43 N-terminally or C-terminally as described above.

Despite knowledge about plasmin substrate consensus sequences such as proposed by Backes et al. 2000, Hervio 2000 et al. or Yuan et al. 2009 it is important to note that plasmin digestion will not only depend on the primary sequence but to a great extent on structural accessibility of the substrate as was demonstrated in EXAMPLE 2 of the present invention showing that although there are several protease consensus sites, the K43 site is particularly stronger cleaved than the other sites identified (see EXAMPLE 2). This shows that not all potential cleavage sites are equally accessible. In addition, substrate recognition is also dependent on post translational modifications.

However, in contrast to changes of neighbour amino acids of K43 or combined changes, the most preferable, substitution K43→A43 is subtle while providing the essential advantage of complete digestion inhibition as demonstrated in EXAMPLE 4 while keeping all desired physicochemical properties for the FceRIa molecule as demonstrated in EXAMPLES 5-7. At the same time this substitution will minimize immunogenicity to protease resistant FceRIa. Taken together, it is preferable to leave the wild type FceRIa structure as "natural" as possible in order not to lose affinity (i.e. ON- and OFF-rates), pH stability and folding properties.

Together, the most preferable substitution is a subtle K43→A43 substitution that does not introduce antigenicity or structural changes of FceRIa..

Although the data of the present invention show that the K43 cleavage site is the most important protease cleavage site, and since there are other protease cleavage sites disclosed or postulated, further amino acid exchanges may be introduced, e.g. to make the FceRIa molecule even resistant to cleavage on these sites or to improve other properties of this molecule. Preferably, a further amino acid is exchanged at an amino acid selected from lysine at position 31 (K31), arginine at position 40 (R40), isoleucine at position 41 (I41), phenylalanine at position 42 (F42), glycine at position 44 (G44), glutamic acid at position 45 (E45), lysine at position 142 (K142), lysine at position 196 (K196), arginine at position 199 (R199), and lysine at position 201 (K201), especially selected from the group consisting of lysine at position 31 (K31), arginine at position 40 (R40), lysine at position 142 (K142), lysine at position 196 (K196), arginine at position 199 (R199), and lysine at position 201 (K201). Of course, also other modifications may be introduced which are known to improve the properties of FceRIa but keep the protease resistance according to the present invention as well as the functional characteristics of FceRIa especially for effectively binding IgE and avoiding introduction of new epitopes and for correct refolding when recycling the apheresis adsorber by denaturation.

The conservation of sequence similarity within a region of up to 8 amino acids flanking the K43 position N- and C-terminally, respectively, shows a structural and functional conservation of this region (see below for partial alignment of FceRIa N-terminal sequences from selected species). Therefore it is preferred to minimize amino acid substitutions within the protease consensus sequence, in particular surrounding up to 4 amino acid positions N-terminal and C-terminal of K43 for inactivating protease susceptibility at this stage. At the same time, however, it is necessary to minimize the risk of forming new epitopes by structural changes or by reducing IgE binding behaviour. In order to minimize this risk, it is preferred to substitute K43 by a hydrophilic/polar/basic or acidic homologue residue as found in other mammalian species such as e.g. present in guinea pig (E; Glutamic Acid) or mouse and rat (T; Threonine). However, the most conservative substitution by R (Arginine) such as found in some non-human primates or pig will not provide effective protease resistance since Arginine is known to be part of a plasmin consensus as further commented below (see e.g. Bakes et al 2000).
Species alignment of N-terminal FceRIa sequences covering the K43 site of human FceRIa:

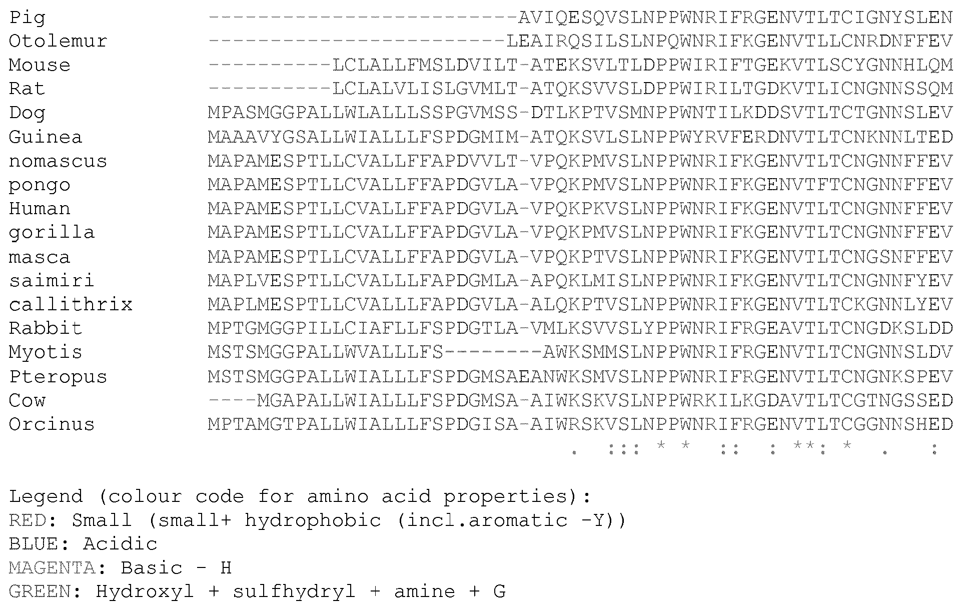

The FceRIa according to the present invention is preferably immobilised on a solid surface so as to enable e.g. surface capturing of IgE. The solid surfaces can be any surfaces able to immobilise polypeptide molecules (e.g. microbeads, microparticles, filters, glass, silicon, metal, metal-alloy, anopore, polymeric, nylon or plastic). Such solid surface materials and methods for binding FceRIa to such materials are well available to a person skilled in the art; all materials and methods used for native FceRIa in the prior art may be used according to the present invention.

According to another aspect, the present invention relates to the FceRIa according to the present invention, for use in the prevention and/or treatment of IgE mediated diseases, wherein the FceRIa is used for depletion of excess IgE (anti IgE therapy) from human body fluids, especially human plasma or serum, in apheresis.

The FceRIa according to the present invention may be used for any medical/therapeutic/diagnostic use suggested and performed for native FceRIa and provides the advantages according to the present invention, i.a. protease resistance and structural nativeness while preserving native folding and full functionality.

Accordingly, the FceRIa according to the present invention may be used for manufacturing a medicament for the prevention or treatment of allergic diseases, preferably seasonal, food, pollen, mold spores, poison plants, medication/drug, insect-,scorpion- or spider-venom, latex or house dust mite allergies, pet allergies, allergic rhinitis and -conjunctivitis, allergic conjunctivitis, allergic asthma bronchiale, non-allergic asthma, Churg-Strauss Syndrome, atopic dermatitis, nasal polyposis, Kimura's disease, contact dermatitis to adhesives, antimicrobials, fragrances, hair dye, metals, rubber components, topical medicaments, rosins, waxes, polishes, cement and leather, chronic rhinosinusitis, atopic eczema, IgE related autoimmune diseases, preferably chronic (idiopathic) and autoimmune urticaria, cholinergic urticaria, mastocytosis, especially cutaneous mastocytosis, allergic bronchopulmonary aspergillosis, chronic or recurrent idiopathic angioedema, interstitial cystitis, anaphylaxis, especially idiopathic and exercise-induced anaphylaxis, immunotherapy, eosinophil-associated diseases, preferably eosinophilic asthma, eosinophilic gastroenteritis, eosinophilic otitis media and eosinophilic oesophagitis; lymphomas, sensibilisation side effects of an anti-acidic treatment, preferably for gastric or duodenal ulcer or reflux.

According to the present invention, an effective amount of the FceRIa according to the present invention is administered to a patient in need thereof (e.g. administered as an injectable, orally or otherwise parenterally delivered therapeutic), especially a human patient (who is in the center of the present therapeutic use anyway).

The advantages of the FceRIa according to the present invention make it specifically suited for the apheresis technique to decrease IgE content in the blood of patients of an IgE related disease. Accordingly, the FceRIa is preferably coupled to a solid carrier which is suitable for contacting with the blood stream of a human individual.

Accordingly, another aspect of the present invention refers to an apheresis device comprising a solid carrier capable of being contacted with the blood or plasma flow, characterised in that the solid carrier includes an FceRIa according to the present invention.

Since proper use of apheresis devices may also require recycling (desorption) that may be conducted by pH shock, preferred adsorbers should be pH resistant, i.e. the FceRIa molecule should refold correctly so that its affinity and specificity is not hampered.

FceRIa according to the present invention may be immobilized effectively on an apheresis matrix support (i.e. the solid carrier), preferably by covalent, oriented/directed immobilization (especially by one defined reactive group on the molecule such as e.g. thiol group of cysteine, primary amine of lysine, artificially introduced aldehydes etc. For functionalization of side groups on proteins, see e.g. review by van Vught et al. 2014 where general applications to any recombinant protein are listed. In the course of immobilization, it is also clear that care has to be taken to prevent negative functional consequences for the immobilised protein to the extent possible. For example, it is clear that functionally relevant cysteines or lysines are sufficiently protected in the course of such immobilization.

The (extracorporal) blood or plasma flow containing IgE to be removed from the blood of a patient having an IgE related disease is conducted over this solid surface to specifically remove IgE by binding IgE to the FceRIa variant molecule according to the present invention on the solid surface of the apheresis device according to the present invention.

The device according to the present invention preferably contains a sterile and pyrogen-free column as a carrier.

According to the present invention apheresis or plasma apheresis is defined as a medical technology in which the blood of a patient is passed through an apparatus that separates out one particular constituent and returns the remainder to the circulation. According to the invention, apheresis is used to separate out IgE molecules from the plasma by use of the FceRIa molecules provided in the present invention. According to the present invention an apheresis device comprises a solid column which can be brought into contact with the blood or with the plasma flux and which has FceRIa variants according to the present invention as receptors that bind IgE. A sterile and pyrogen-free column is preferably used as apheresis carrier. Herein, "column" is defined as a module of any shape having a matrix material to which proteins can be chemically coupled.

Preferably, the matrix material of the solid carrier is a carbohydrate based material such as Sepharose™, dextrane, agarose or cellulose. Other suitable matrix materials include autoclavable matrices such as beads, fibres and membranes or films composed of glass or synthetic polymers such as polymethacrylates, polystyrenes and polyamides. In cases where beads are used, the diameter of the beads is not limited as long as the liquid phase of the apheresis can circulate. However to reduce the flow resistance, those beads having a diameter of 50 to 3000 µm, especially 200 to 3000 µm are preferably used. The matrix material is sterilised by pre-rinses with a sterile solution and additional steam treatment at low temperature according to US 5,817,528 A or any other technique known to the skilled artisan. The sterile and pyrogen-free matrix material is then activated by incubation with cyanogen-bromide solution as described in US 5,817,528 A or any other technique known to a person skilled in the art. Successively the desired antigen-binding molecules are coupled to the column by incubation. Alternatively activation and coupling can also be achieved simultaneously by incubation of the antigen-binding molecules together with 1,1'carbonyldiimidazole according to US 5,817,528 A or any other technique known to a person skilled in the art. Once the coupling procedure is finished, the matrix material having antibodies coupled thereto is extensively washed and tested for cyanate ester, sterility and pyrogenicity. The amount of the adsorber molecule to be immobilised in the column and the size of the column are not restricted. The coupled matrix material is also tested for total bound protein, and binding activity of the coupled protein. The coupled matrix material is then filled under aseptic conditions into sterile, depyrogenated, silanized glass housings to form sterile and pyrogen-free protein-coupled columns. The flow rate through the apheresis device may be controlled by appropriately selecting the inner diameters of the tubes of the circuit or by using an auxiliary pump (US 4,770,774 A). The column of the invention can be repeatedly used and recycled by eluting the absorbed IgE after use.

The apheresis device according to the present invention may comprise further IgE binding molecules (such as antibodies, native FceRIa or other known IgE binding molecules), if appropriate; however, due to the protease resistant character of the present FceRIa molecules, it is most preferred to use these molecules as the only kind of molecules (or together with other protease-resistant IgE binding molecules, if available).

The present invention also relates to the use of an apheresis device according to the present invention for providing a prevention and/or treatment device for preventing and/or treating an IgE related disease, especially for performing an anti IgE therapy.

According to another aspect, the present invention relates to a kit for use in preventing and/or treating IgE related diseases comprising a solid apheresis carrier containing FceRIa according to the present invention, wherein said carrier is a sterile and pyrogen-free column.

The kit may further comprise connecting and conveying means to the patient to receive blood from the patient and to bring the blood back to the patient (with decreased amount of IgE), pumping means for pumping blood or plasma over the solid surface with the FceRI variants according to the present invention and suitable controlling devices for controlling the blood or plasma stream and the apheresis performance (adsorption, etc.) before, during and after the treatment of the patient (e.g. also afterwards in the course of regeneration of the apheresis column).

The present invention also relates to FceRIa according to the present invention for use in a therapeutic method, especially for use in the prevention or treatment of IgE related diseases, and/or for use in the manufacture of a medicament for the prevention or treatment of IgE related diseases.

The present subject matter is specifically suited to be combined with a further IgE lowering therapies, for example the anti-IgE therapies listed supra under "Anti-IgE therapies and limitations", especially for example the combination therapy disclosed by Kerzel et al., 2011, applying an anti-IgE antibody (such as Omalizumab^{®}) with plasmapheresis.

Although the use of the present FceRIa variants for IgE apheresis is a specifically preferred embodiment, the FceRIa molecules according to the present invention may be used for any of the (therapeutic or diagnostic) purposes suggested and performed in the prior art for native FceRIa or compositions comprising native FceRIa. For example, FceRIa according to the present invention may be used as an administered biological therapeutic (e.g. by injection, oral delivery or any other parenteral routes used for delivery of biological therapeutics, such as i.v., i.m., s.c., pumps, transdermal, inhalative, etc.). Accordingly, the present invention also relates to a pharmaceutical composition comprising FceRIa according to the present invention and a pharmaceutically acceptable carrier.

Preferably, the pharmaceutical composition according to the present invention further comprises an agent which increases the half-life of the FceRIa variant according to the present invention in a patient to whom the composition is administered. Examples for such half-life increasing agents are known to a person skilled in the art; examples are human serum albumin (HSA) or the introduction of PEG groups ("PEGylation").

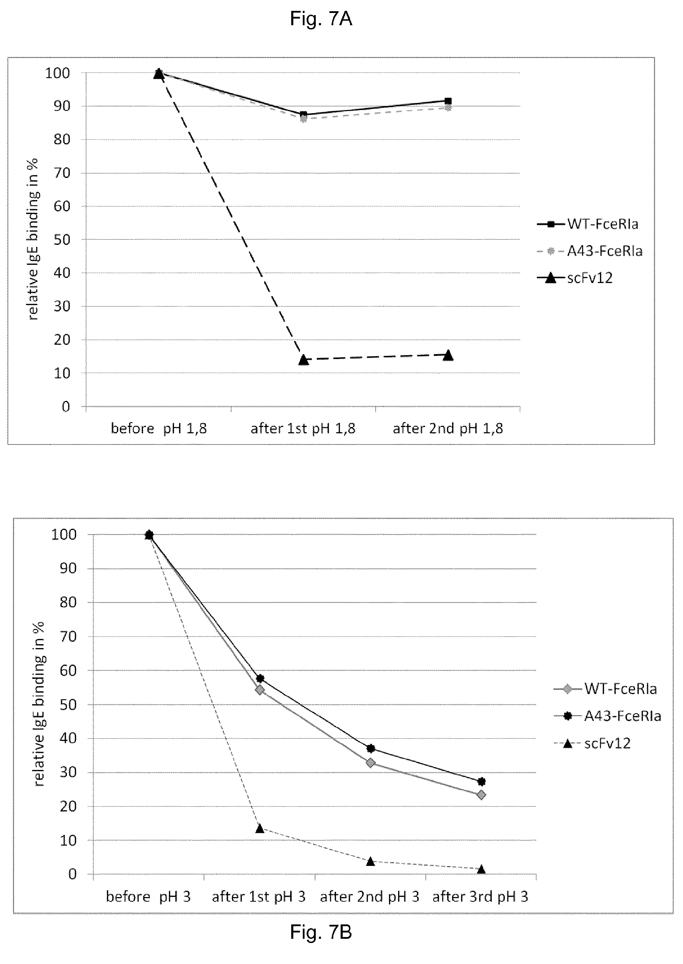

According to another aspect, the present invention relates to the use of the FceRIa according to the present invention as a recyclable probe for IgE detection in protease containing samples, especially plasma samples or IgE- and membrane IgE containing tissues and cells. The protease resistant FceRIa variant according to the present invention can for example be re-cycled by denaturation/renaturation when samples have to be measured sequentially instead of parallel, such as in a typical Biacore setting. As for apheresis, the importance or re-usability after denaturation is also relevant in such probe uses. The probe according to the present invention is not harmed by proteases in the samples or by following recycling steps including denaturation/renaturation. Once IgE has been trapped and measured, IgE it can be released by denaturation without harming the FceRIa according to the present invention. After renaturation (without loss of functionality in contrast to e.g. scFv12), the next measurement can be performed as demonstrated in the following examples (see e.g. FIGURE 7A).

Application of the present protease resistant FceRIa as a probe for detecting IgE (preferably from plasma and all other protease containing samples) can be applied in any capture/detection based method. Accordingly, it is specifically preferred to use the present probe in an ELISA (enzyme-linked immunosorbent assay) or SPR (Surface Plasmon Resonance; Biacore) assay.

Application of the present protease resistant FceRIa as a molecular imaging tracer in combination with a fluorophore allowing for the in vivo detection of IgE or membrane IgE-containing tissues or body fluids and compartments for the purpose of diagnostics or biomarker analysis represents a preferred use of the present invention. Labelled FceRIa represents a relatively small tracer well suited for optical in vivo imaging. In general, smaller tracers provide advantages for molecular imaging such as reviewed in detail by Oliveira 2015.

Moreover, the present protease resistant FceRIa can be provided as part of a more sophisticated (poly-) functional construct as modern therapeutic, for example as part of bifunctional mABs or some other state-of-the-art biologicals that combine several functionalities due to their modular system: Accordingly, the protease resistant FceRIa according to the present invention can also be used as part of a conjugate or polyfunctional construct for intra- or extracorporal therapeutic and diagnostic or imaging purpose. As a recombinant fusion protein, it can be part of a bi- or polyfunctional protein whereby, according to the common art, functional domains can be genetically fused to each other via inert flexible or rigid peptide linkers such as reviewed by Chen et al. 2013. Such linkers and attached domains will have an impact on stability, solubility, expression yields, biological activity, antigenicity and pharmacokinetics. Such linkers can also be designed such that they are cleavable in vivo in order to achieve controlled activation or delivery to a target cell or tissue e.g. for local activation of a therapeutic function or for providing specific label activation in situ such as needed for in vivo imaging.

A prototypic example for a functional domain that can be combined with FceRIa is human serum albumin. This was initially proposed by Digan et al. (Patent WO 1998/004718 A1). As preferred alternatives, transferrin or Fc domains can also be used to modulate pharmacodynamic behaviour of biologicals. According to the common art, FceRIa can be genetically or chemically linked or heterodimerized to other functional protein or peptide entities such as e.g. cytokines (commonly termed "immunocytokines", as reviewed in Bootz & Neri 2015) or to engineered antibodies, antibody fragments or antibody-like structures as extensively reviewed by Spiess et al 2015. Emerging formats for the design of bi- or polyspecific therapeutics include (as examples for anitbodies, antibody fragments or antibody-like structures) nanobodies, (tandem-)scFv's, (tetravalent bispecific tandem-)IgG, dual targeting domains, BiTE's, Triomab's, DART's, DVD-Ig, IgG-fynomers etc. Extensive reviews for common formats currently in clinical evaluation are provided by Spiess et al. 2015. Based on their technical flexibility, such formats could be used as fusion proteins with FceRIa in order to provide and combine new therapeutic functionalities to FceRIa. Accordingly, the FceRIa according to the present invention may be coupled to (one or more of each of the following groups of functional molecules):
- a linker molecule, preferably a peptide linker, especially a peptide linker consisting of one to ten, preferably two to five, amino acid residues; and/or
- a therapeutic or diagnostic molecule, preferably a monoclonal antibody or antibody fragment, a cytokine, an antibody-like structure, a cytotoxic agent preferably a toxin or a cytotoxic drug, an optical tracer; and/or
- a carrier, preferably a carrier protein, especially human serum albumin, transferrin or a Fc domain.

According to a preferred embodiment of this aspect, the protease resistant FceRIa according to the present invention can be used in a similar manner than an antibody drug conjugate (ADC) therapeutic for targeting IgE B-cell receptor expressing cells such as IgE-switched B-cells. This will kill or arrest these cells or provide inhibitory signalling ultimately resulting in IgE lowering. ADC's are typically used as anticancer drugs for delivering functionally active, notably cytotoxic agents to specific target cells as reviewed by Peters & Brown 2015. Accordingly, the FceRIa according to the present invention is chemically coupled or genetically linked to at least one cytotoxic agent. This coupled molecule is then suitable to target e.g. IgE B-cell receptor expressing cells thereby enabling IgE lowering such as needed for the treatment of allergy or other IgE related diseases as mentioned supra based on the fact that killing or suppressing IgE B-cell receptor expressing cells will ultimately reduce the number of IgE producing plasma cells and thereby soluble IgE levels in the patient. Alternatively, any other cells expressing the IgE B-cell receptor such as rare cancer forms could also be targetted by an FceRIa Drug Conjugate in analogy to an Antibody Drug Conjugates approach. The combination of the FceRIa according to the present invention and such further (functional) molecules can preferably regarded as an ADC-like entity (as defined by Peters & Brown, 2015) comprising the FceRIa according to the present invention for targeting cytotoxic agents specifically to IgE B-cell receptor expressing cells.
Fig. 1 shows co-precipitation of plasmin by FceRIa.
Fig. 2 shows incubation/digestion of recombinant FceRIa with pooled human sera (A) and with plasmin (B).
Fig. 3 shows that IgE depletion is reduced to a different extent when incubating wild type or protease resistant A43-FceRIa adsorber with plasmin.
Fig. 4 depicts a Base Peak Chromatogram showing the complete absence of a cleavage product in the mutant, protease resistant variant of recombinant A43-FceRIa (black line), whereas the wild type receptor digest peak appears at 30min (grey line).
Fig. 5 shows that calculated EC50 values (24.5ng/ml, 34,4ng/ml and 42,8ng/ml for FceRIa, A43-FceRIa and ScFv12, respectively) and curve shapes of IgE binding to A43-FceRIa are similar to wt-FceRIa or scFv12 demonstrating no negative effect of the K→A change at position 43 of the FceRIa sequence.
Fig. 6 shows that IgE could be almost completely desorbed from FceRIa by pH 3.4 treatment (upon equal immobilization of FceRIa and scFv12 onto beads followed by in vitro IgE adsorption), whereas IgE was not released from ScFv12.
Fig. 7 shows regeneration performance with or without serum; the stabilizing/renaturing effect of serum milieu for the protease resistant FceRI adsorber is reflected by moderate reduction in IgE-binding capacity with serum regeneration (Fig. 7A) compared to conditions without serum regeneration (Fig. 7B).

### EXAMPLES

The present invention provides an improved variant of FceRIa that combines two essential features: First, cleavage at a newly recognized proteolytic cleavage site within the N-terminal portion of FceRIa is excluded while at the same time, the structure of FceRIa is not changed in order to preserve IgE binding, pH stability and refolding characteristics (as demonstrated in EXAMPLES 5, 6 and 8). In order to render FceRIa protease resistant, it is particularly important to avoid the formation of new epitopes that are not normally present within the wild type FceRIa sequence. Besides protease resistance in blood, plasma or serum, the modification of the present invention must guarantee no or only minimal structural changes in order to minimize the risk of *de novo* immunogenic sites. At the same time it should preserve the same advantageous desorption and refolding characteristics that wt FceRI can provide (as demonstrated in EXAMPLES 5, 6 and 8).

A physical association between FceRI and plasma proteases was found in EXAMPLE 1. The identification of a plasma protease-sensitive site in the N-terminal portion of FceRIa is demonstrated and explained in EXAMPLE 2. EXAMPLE 3 shows that proteolytic cleavage of recombinant FceRIa leads to the reduction of IgE binding which is detrimental for IgE inhibition (e.g. when used as a therapeutic IgE blocker) or for extracorporal IgE removal (e.g. when used as selective IgE adsorber). In this example it is shown that cleaved FceRI adsorber suffers loss of adsorption efficacy which is corroborated by previous functional/structural studies showing that the N-terminal domain of FceRIa contains portions that are required for effective IgE binding (Mallamaci et al. 1993). Based on the present finding of a new protease sensitive site (designated K43) and the assessment of its functional relevance for IgE binding, the present invention provides a technical solution that renders the FceRIa adsorber protease resistant to protease digestion at K43 allowing for sustained contact with blood, plasma or serum as demonstrated in EXAMPLES 4 and 5. EXAMPLES 5 and 6 demonstrate that protease resistant FceRIa has comparable IgE binding- and adsorbing qualities when compared to prior art scFv12 (Lupinek et al. 2009 and US 2014/124448 A1). However in contrast to scFv12, the protease resistant FceRIa adsorber shows significantly higher pH stability than scFv12 allowing for desorption/renaturation and recycling when used e.g. in apheresis. EXAMPLES 7 and 8 show that the prior art single chain antibody adsorber scFv12 cannot be recycled after IgE desorption because of its destruction at low pH. However in order to allow multiple use of one column to save cost, it is important to provide an apheresis column that allows desorption (e.g. by low pH) and regeneration *without* irreversibly damaging or denaturing the adsorber molecule as demonstrated in EXAMPLES 8. Eventually the protease resistant FceRIa adsorber of the present invention is also capable of depleting anti FceRI autoantibodies by therapeutic apheresis. Such autoantibodies can be found in conditions such as e.g. chronic autoimmune urticaria (Zuberbier et al. 2000). This feature is not provided by IgE adsorbers that are based on anti IgE antibodies or derivatives of antibodies such as e.g. ScFv's or Fab's. Most important the present invention provides a subtle modification of the FceRIa sequence that does not alter IgE binding- and refolding of the molecule. The risk of presenting a "non-self", new antigenic structure to the immune system is minimized by the fact that one single, small and uncharged amino acid can be used for complete abolishing of protease cleavage. Therefore, the present protease resistant FceRIa can be used not only for extracorporal therapeutic treatments such as e.g. selective IgE apheresis or depletion of autoantibodies but also as a basis for generating an administered (e.g. by parenteral, oral or other commonly used administration routes) therapeutic for passive administration into the blood stream.

In summary, the present invention relates to an artificial modification of the FceRIa chain that renders it resistant to digestion by plasma proteases while preserving its functionality and pH stability and while minimizing the risk for immunogenicity induced by "foreign" amino acid substitutions that carry the risk of modifying the natural FceRIa structure. The new protease-resistant FceRIa variant thus provides an advantage for the use of the FceRIa chain in therapeutic applications in which IgE binding is required.

### EXAMPLE 1: Association of FceRIa with plasma proteases.

Recombinant FceRIa co-precipitates with several plasma proteins including serum proteases such as plasminogen or prothrombin. This association renders it possible that FceRIa is cleaved by these proteases during the contact with blood, plasma or serum. TABLE 1.1 lists the proteins that were identified in a co-precipitate of recombinant FceRIa incubated with human serum as explained below under *Material* & *Methods.*

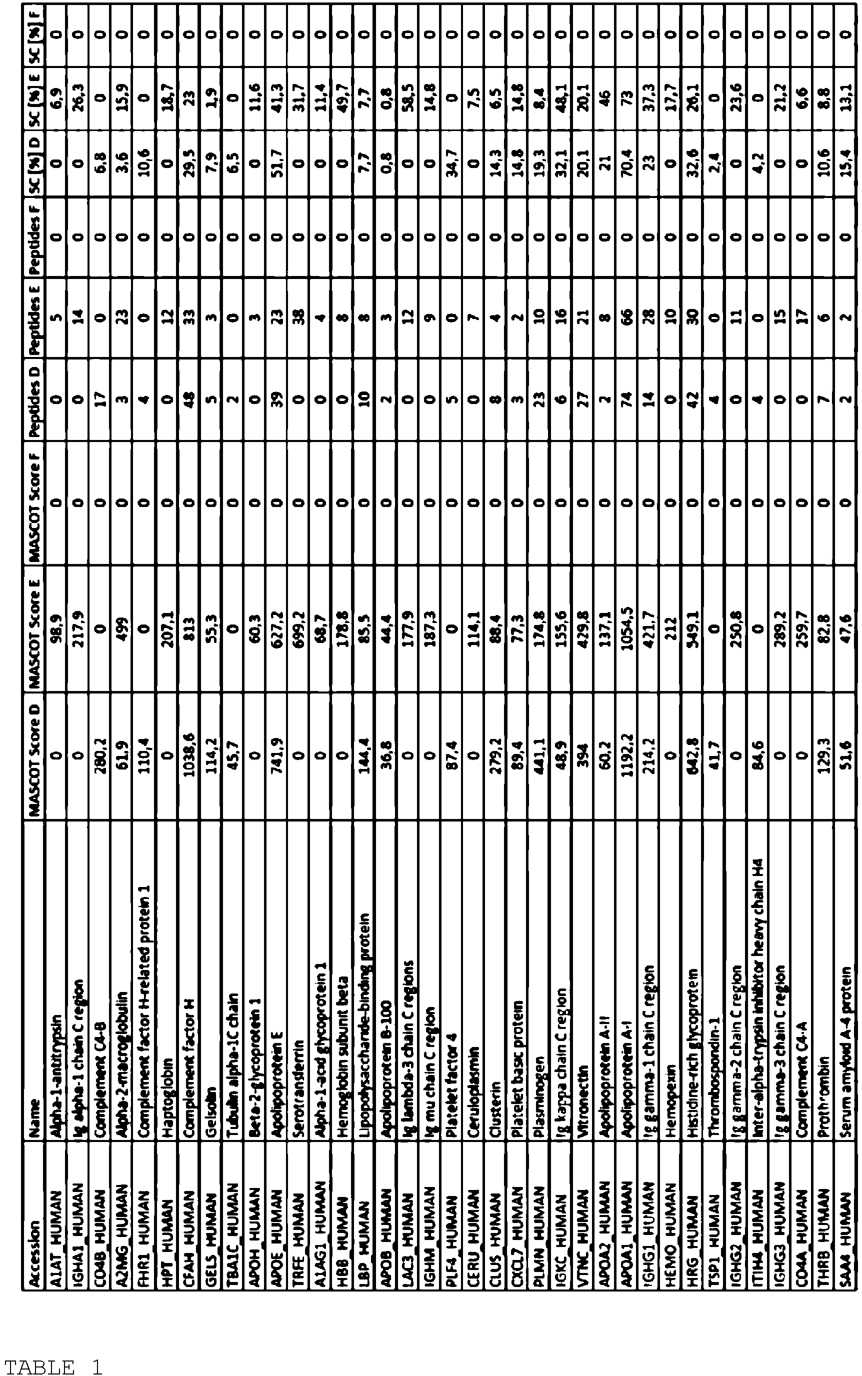

As a result, at least two relevant proteases, namely plasminogen/plasmin and prothrombin/thrombin associate with recombinant FceRIa upon incubation with human serum. This provides the possibility of unwanted proteolytic cleavage and degradation of FceRIa protein when administered into the blood or applied as an adsorber in apheresis. Results from EXAMPLES 2 and 3 demonstrate that FceRIa is proteolytically cleaved which is indeed detrimental to IgE binding.

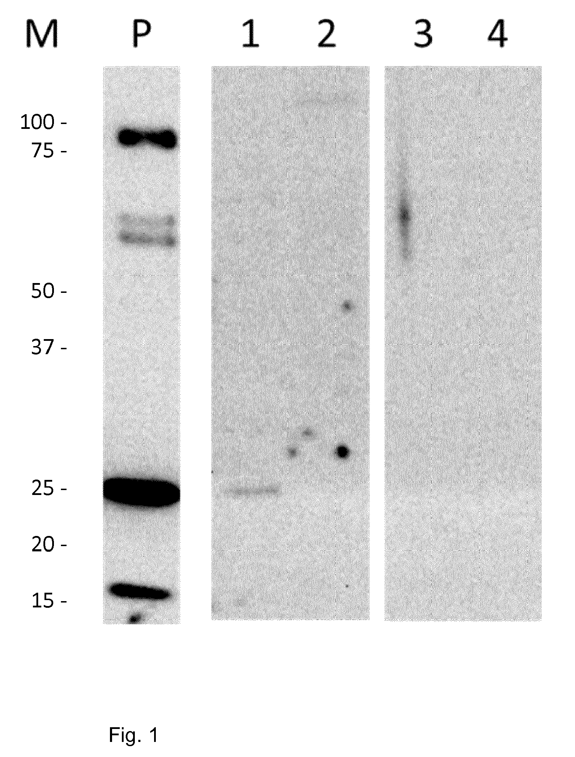

FIGURE 1 shows the co-precipitation of plasmin (Sigma P1867) by FceRIa upon incubation at 10µg/ml provides evidence for direct physical interaction of the adsorber with plasma protease (s) as identified with serum incubation (see TABLE 1). Co-precipitated plasmin was resolved by Western Blot analysis; lanes were loaded as follows: M, protein marker; lane P, plasmin; lane 1, FceRIa (50µg on beads) + 1µg Plasmin; lane 2, FceRIa + 100ng Plasmin; lane 3, HSA + 1µg Plasmin; lane 4, HSA + 100ng Plasmin. Consistent with the results from the serum co-precipitation experiment, co-precipitated plasmin can be revealed as a 25kD band when using recombinant FceRIa-coated beads (lane 1) as opposed to control beads coated with HSA (lanes 3 and 4). The association *in vitro* between FceRIa and plasmin corroborates the results from serum co-precipitation as shown in TABLE 1 (above).

### Material and Methods:

### Expression and purification of recombinant FceRIa:

HEK293 freestyle cells (Invitrogen R790-07) were grown at 37°C under rotation (180rpm) in 125ml and 250ml flasks in appropriate medium (Invitrogen 12338-026). Cells were maintained by splitting each second day 1:3 to 1:5 allowing for maximal cell density of 2x10E6 cells /ml. For transfection, cells were split 24h at 5x10E5 cells /ml before treatment in a volume of 30ml medium with 30µg Plasmid DNA, 30µl MAXreagent (Invitrogen 16447-100) and 1ml Optimem (Invitrogen 31983-062). Recombinant eukaryotic FceRIa was cloned between EcoRI and BamHI of eukaryotic expression vector pTT5 (NRC National Research Council Canada). The construct was composed of a signal peptide derived from Genbank Seq BAA75054.1 followed by UniProt Sequence P12319 [FCERA_HUMAN] starting from amino acid 20 (all numberings in this document according to UniProt Sequence P12319) until amino acid 205 covering the extracellular region of FceRIa followed by a spacer and 6His for standard Ni-NTA purification. After pre-washing, 1ml of Ni2+ NTA Agarose beads (Qiagen 30210) and 2-5ml of pre-concentrated cell culture supernatant was applied to a 2ml Talon gravity column (Takara 635696) and washed several times with 6xHIS WASH BUFFER (50mM Phosphate Buffer, 300mM NaCl, 5mM Imidazole, pH 8). Elution was performed with 6xHIS ELUTION BUFFER (50mM Phosphate Buffer, 300mM NaCl, 150mM Imidazole, pH 8) under non-denaturing conditions (according to the manufacturer Protocol Qiagen 30210). The eluate was concentrated to 100-200µl using AMICON-ULTRA-15-Centrifugal Filters (10K) (Millipore; Cat-No: UFC801024), washed 3x with 6ml PBS and concentrated again to 100-150µl and used for immunoprecipitation.

### Detection of co-precipitated plasma proteases upon incubation of recombinant FceRIa with human serum.

Adsorber-coated beads (Bioclone FG-102) were incubated with either 50µl of a mix of 17 human sera, or 50µl of a IgE-depleted serum mix or with 50µl PBS for 21h at 27°C while shaking at 900rpm. After incubation, beads were washed 3x with PBS /0.1% BSA /0.5% Tween and 3x with PBS.

Beads with immobilized protein were mixed with ammonium bicarbonate buffer (ABC buffer; Acros organics Cat.# 1066-33-7) containing 15 mM DTT (Roth, #6908.3) and incubated for 45 min at 56°C to reduce disulphide bonds. Iodoacetamide (Sigma # I1149-5G) in 100 mM ABC was added and the mixture was incubated for 30 min in the dark for carbamidomethylation of Cysteines. Beads were washed twice with 100mM ABC buffer and incubated over night with 5µg Trypsin (Promega #90058). Digested samples were loaded on a BioBasic C18 column (BioBasic-18, 150 x 0.32 mm, 5 µm, Thermo Scientific) using 0.1% formic acid as the aqueous solvent. A gradient from 5% B (Solvent A: 0.1% FA in water, 0.1% FA in ACCN) to 32% B in 35 min was applied, followed by a 15min gradient from 32% B to 75% B that facilitates elution of large peptides, at a flow rate of 6 µL/min. Detection was performed with a QTOF MS (Bruker maXis 4G ETD) equipped with the standard ESI source in positive ion, DDA mode (= switching to MSMS mode for eluting peaks). MS-scans were recorded (range: 150-2200 Da) and the 6 highest peaks were selected for fragmentation. Instrument calibration was performed using ESI calibration mixture (Agilent). Data files were converted (using Data Analysis, Bruker) to mgf files, which are suitable for performing a MS/MS ion search with GPM. GPM is a web-based, open source user interface for analyzing and displaying protein identification data (http://human.thegpm.org). The interface creates a series of web browser page views of tandem mass spectrometry data that has been assigned to protein sequences. Among the identified proteins, two proteases (i.e. prothrombin and plasminogen) with theoretical cleavage sites identical to trypsin where identified (see Perkins et al. 1999). Since a tryptic digest could have interfered with the approach to identify protease cleavage sites, an alternative digest (GluC, Roche, # 1104781700) was performed to release the peptides. TABLE 1 displays the identified proteins with their MASCOT scores http://www.matrixscience.com/help/scoring help.html), respectively. The higher the MASCOT scores are, the better the accordance of the detected proteins with the theoretical protein spectra. Scores were ranked against the PBS sample (MASCOT score 3). (See Botelho et al. 2010 for more detailed references about MS methods.).

### Co-precipitation of plasmin:

FceRIa coated beads (Bioclone FG-102) were incubated with either 50µl human serum mix (as in EXAMPLE 1) or in 50µl PBS for 21h at 37°C while shaking at 900rpm followed by 4x washing with PBS. Protocol of Reduction, S-Alkylation, GluC digest and the mass spectroscopic analysis was performed as in EXAMPLE 1. Recombinant FceRIa protein was coupled to 1µm BcMag Iodoacetyl-activated magnetic beads (Bioclone FG-102) at 1mg Protein/ml beads according to the manufacturer's protocol. After blocking with 8mg/ml Cysteine, beads were washed 3x with PBS + 0.5% Tween. 1x Plasmin Digest Buffer (0.1M Tris-HCl, 2mM EDTA, pH 8) was added to the beads followed by incubation at 37°C for 1hr at indicated Plasmin concentrations. For Western blot analysis, beads were washed 3x with PBS and 3x with PBS + 0.1% Tween before adding LDS Buffer (Invitrogen Cat#NP0007) and sample reducing agent (Invitrogen Cat#NP0009). After denaturing for 10min at 95°C, samples were loaded on NuPAGE Novex 4-12% Bis Tris Gel (Invitrogen NP0322BOX) and run 2h/70V. Gels were blotted to membranes using QUICK-TRANSFER SYSTEM (Biorad, settings: Standard, mixed MW, 1,3A, 25V, 7min) and blocked with 1% non-fat milk in PBS-T (1xPBS, 0,1% Tween 20) 2h, RT. Detection of plasmin was performed with 1:5000 mouse anti Plasminogen (R&D System, MAB1939) in PBS-T by incubating the membrane ON at 4°C. After washing 30min with PBS-T, 1:10000 diluted anti-mouse HRPO (Jackson, Cat#115-035-068) was added in TBS-T for 2h, followed by final washing in TBS-T and detection by Clarity Western ECL Substrate (BioRad 170-5060). Membranes were exposed to detection screen (UVP BioImaging Systems, AutoChemi System) between 1-10minutes.

### EXAMPLE 2: Cleavage of FceRIa by a plasma proteases.

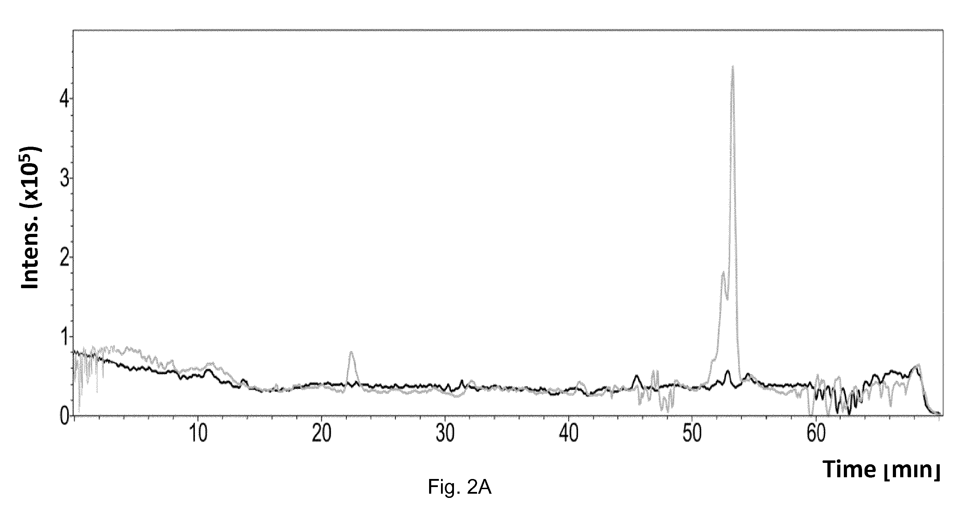

FceRIa (numbering based on the UniProt sequence P12319) was cleaved upon incubation with serum and plasmin. Protease sensitivity was observed when incubating recombinant FceRIa with pooled human sera derived from patients with various inflammatory conditions but not when incubated with PBS. A strong digestion peak at K43 was identified (FIGURE 2A). Plasmin incubation (TABLE 2.1 and TABLE 2.2) provided evidence for plasmin as a preferential candidate plasma protease and identified K43 as the most sensitive protease cleavage site of FceRIa sequence when compared to other cleavage sites identified.

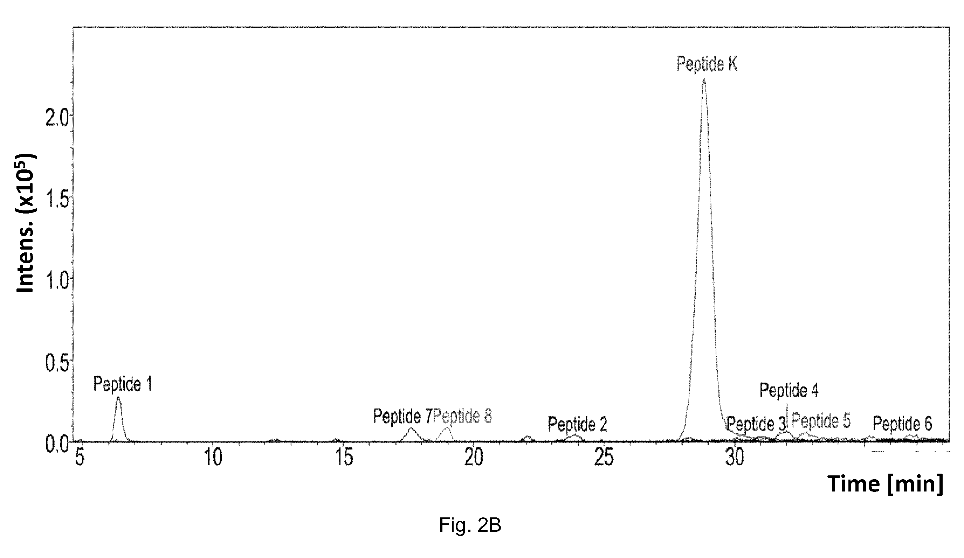

Site K43 (indicated as peptide K in FIGURE 2B) of recombinant wt FceRIa was preferentially cleaved whereas plasmin cleavage sites K31, R40, K201, R199, K196 and K142 (indicated as peptide 1-8 in FIGURE 2B) were cleaved to lesser extent when incubating with plasmin for 1h at 37°C as shown in FIGURE 2B. K43 is therefore defined as a paramount plasma protease hypersensitive site of FceRIa as summarized in TABLE 2.1 and TABLE 2.2. Importantly, the N-terminal region (containing the protease sensitive K43 site) was previously shown to be relevant for proper IgE binding by FceRIa as described e.g. by Mallamaci et al. 1993 who in their study used various deletion and chimaeric receptor variants to provide structure/function insights for FceRIa. FIGURE 2A shows that serum incubation of recombinant FceRIa yielded a digestion product (grey line) in comparison to untreated FceRIa (black line), as shown in the Base Peak Chromatogram (x-axis =time; y-axis=peak intensity). Thus digestion of FceRIa occurred only in the presence of human serum but not PBS. FIGURE 2B demonstrates that this serum protease activity is contributed at least in part by plasmin since upon treatment of recombinant FceRIa with 12.5µg/ml plasmin, it could be shown that unexpectedly, K43-cleavage associated "peptide K" (as indicated in the FIGURE 2B) was more sensitive to protease cleavage than the other residues (such as e.g. Peptides 1-8) since it provided a higher peak signal in the Base Peak Chromatogram. Although the other eight identified plasmin cleavage sites consistently carried plasmin substrate recognition consensus sequences according to prior art knowledge (see for example Backes at al. 2000, Yuan et al 2009 or Hervio et al 2000), they were released to significantly lesser extent when compared to "peptide K" upon plasmin digestion. It was therefore not predictable which of the 9 identified plasmin cleavage sites should be modified in order to render FceRIa serum protease and/or plasmin resistant. To modify simultaneously several plasmin cleavage sites would be possible but not desired since a FceRIa variant with multiple mutations will affect structure and functionality of the receptor.

In order to improve FceRIa plasma or serum-protease stability, it is therefore necessary to introduce amino acid changes that significantly reduce substrate site recognition by proteases such as plasmin in agreement with previously identified substrate specificities for plasmin such as described by Yuan et al. 2009 or by Backes et al. 2000. However it is most important to reduce substrate site specificity for proteases at the most sensitive and relevant cleavage site identified in the present invention, namely K43, as described in EXAMPLE 2. It is also possible but less preferred to substitute alone or in combination the less sensitive plasmin cleavage sites identified including K31, R40, K142 and K196, R199, and K201. In general changing lysine at position 43 (i.e. K43) to any other amino acid (except for arginine and histidine which belong to substrate site recognition motif of plasmin) can block plasmin cleavage susceptibility at K43 since it is known that plasmin cleavage requires a positively charge amino acid. However since *any* substitution of a positively charged amino acid at a plasmin cleavage site increases the risk of perturbing FceRI folding or IgE affinity, it is most preferable to introduce a modification that will *not* affect FceRIa function and recyclability. It is possible to mutate several residues of one substrate recognition sequence simultaneously; however it is preferred to minimize a *priori* structural changes that might carry the risk of undesired neoepitopes or structural changes that can be unintentionally introduced. It is also not desired to introduce foreign amino acids that would allow for undesired addition of *O*-linked or *N-*linked oligosaccharides.

Therefore the most preferred substitution by alanine (A43) was chosen in order to minimize the risk of undesired immunogenicity. In order to maintain A43-mutated, protease resistant FceRIa (designated A43-FceRIa) as "natural" as possible with respect to its protein structure and physicochemical properties, A43 was selected as the most preferred substitution rendering FceRIa plasma/serum-protease resistant as demonstrated in EXAMPLE 2. Since the alanine substitution will *not* introduce unexpected structural changes that might provide undesired immunogenicity, difficulties in immobilization or changes in IgE binding. As a negative example, a cysteine might cause unexpected inter- or intramolecular disulphide bridges whereas a proline might cause structural artifacts due to flexible kink formation in this region. An Arginine substitution for example, will provide at least partial digestion since it corresponds to the substrate recognition site of plasmin or other proteases (see e.g. Backes et al 2000). EXAMPLE 8 demonstrates the necessity and importance of proper refolding after desorption by low pH.

Plasmin cleavage sites are not only dependent on the presence of lysine or arginine (consistent with the species comparison depicted above) or occasionally histidine at the cleavage position, but also on a substrate recognition sequence that was previously explored by Backes at al. 2000 and Yuan et al 2009. As an example, Lys at position -3, tyrosine, Tryptophan or Phenylalanine enrichment at position -1 (which is consistently found according to the homology alignment above) in combination with the essential positively charged amino acid at the position the plasmin cleavage site could constitute a consensus for substrate recognition provided that the site is structurally accessible by the protease. Yuan et al. 2009 further proposed that 69% of the amino acids at position -3 of the plasmin cleavage site were non polar (Backes et al. proposed lysine, valine, isoleucine or phenylalanine at these positions), 85% of the amino acids at position -2 were polar and 81% at position -1 were non polar. In addition, Hervio et al. 2000 suggested that valine might be enriched at position -2 whereas serine was enriched at position +1 suggesting that such substitutions would not be favourable within the K43 cleavage consensus of FceRIa. Protein structure, cleavage site accessibility and nearby post-translational modifications such as glycosylations play an essential role for susceptibility to protease cleavage. This might explain why according to the present invention, K43 is the most sensitive plasmin cleavage site as demonstrated in TABLE 2.2.

Based on the result from EXAMPLE 3, K43 has turned out to be the practically most relevant plasmin cleavage site in contrast to several other, less relevant plasmin cleavage sites listed above. Therefore the major claim of the present invention is to render the K43 cleavage site protease insensitive. At the same time, structure and function of FceRIa must be maintained without reducing the structural and functional qualities of FceRIa. When using protease-resistant FceRIa either for extracorporal IgE depletion by therapeutic apheresis or for passive administration into the blood stream, it is important to avoid (neo-) antigenic sites or functional changes that reduce IgE binding.

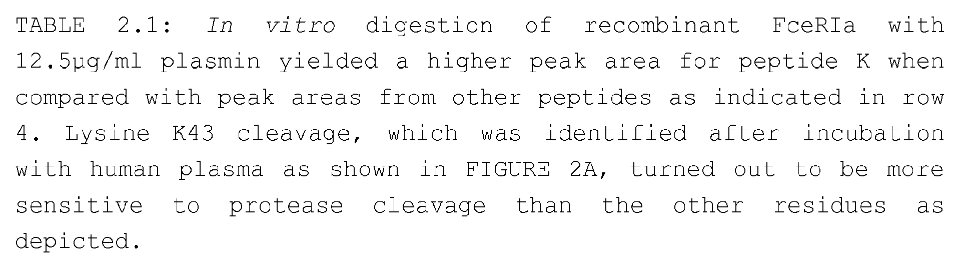

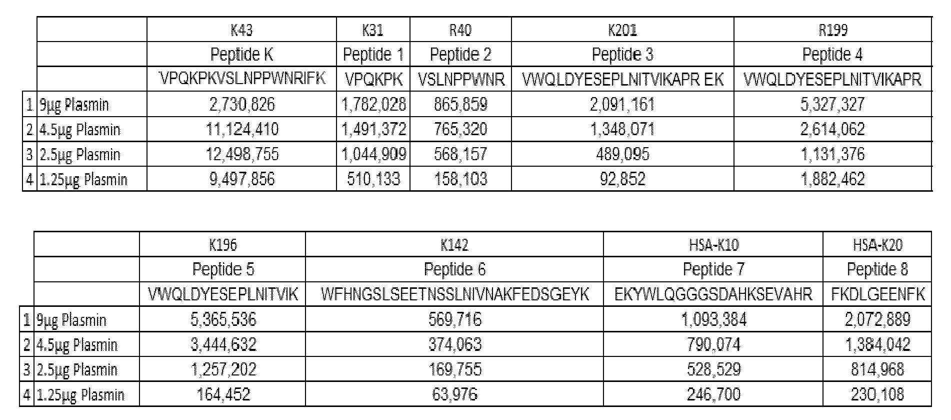

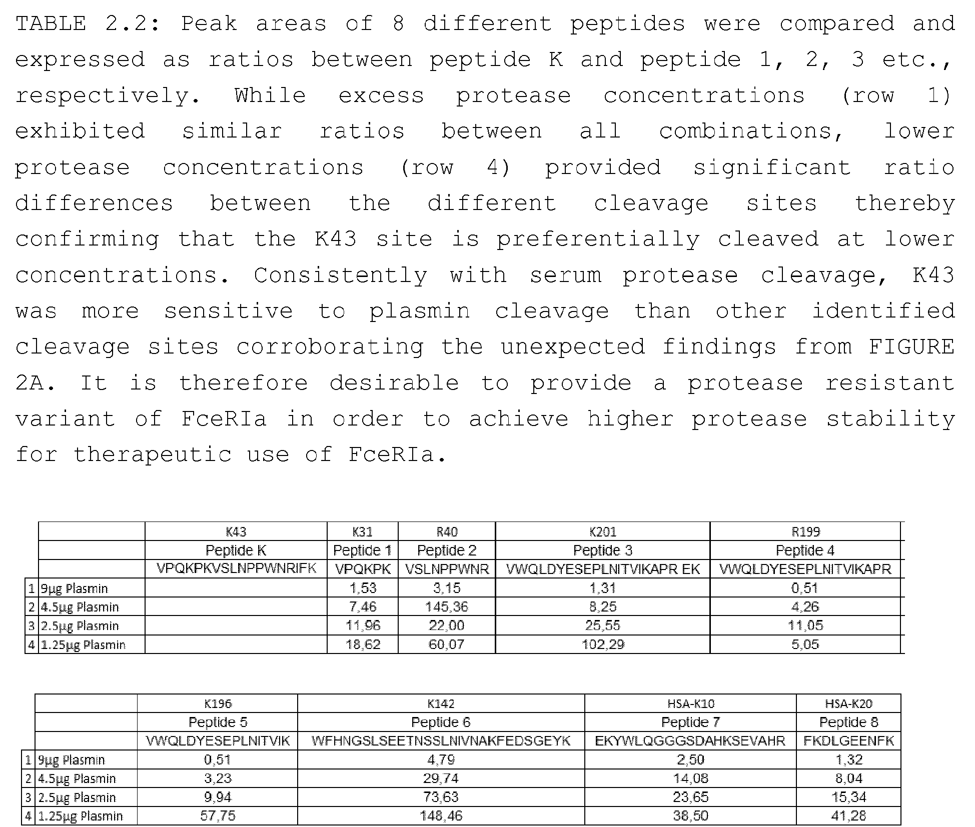

### Material and Methods:

FceRIa coated beads (Bioclone FG-102) were incubated with either 50µl human serum mix (as in EXAMPLE 1) or in 50µl PBS for 21h at 37°C while shaking at 900rpm followed by 4x washing with PBS. Protocol of Reduction, S-Alkylation, GluC digest and the mass spectroscopic analysis was performed as in EXAMPLE 1.

### In vitro plasmin digestion.

Recombinant FceRIa protein was deglycosylated by overnight incubation at 37°C with 0.125 Units PNGase F (Roche #06538355103) in 50 mM NH4Ac Puffer ~pH 8. The protein solution was brought to a final concentration of 1µg/µL by dilution with plasmin reaction buffer (0.1M Tris-HCl, 2mM EDTA, pH 8). The reaction mixtures were incubated at 37°C for one hour at four different plasmin concentrations (1.25 - 9 µg). The digestion was stopped by heat inactivation at 99°C for 5min. Sample loading on a BioBasic C18 column and mass spectroscopic analysis was performed as in EXAMPLE 1.

### EXAMPLE 3: Reduced IgE binding to protease digested FceRIa.

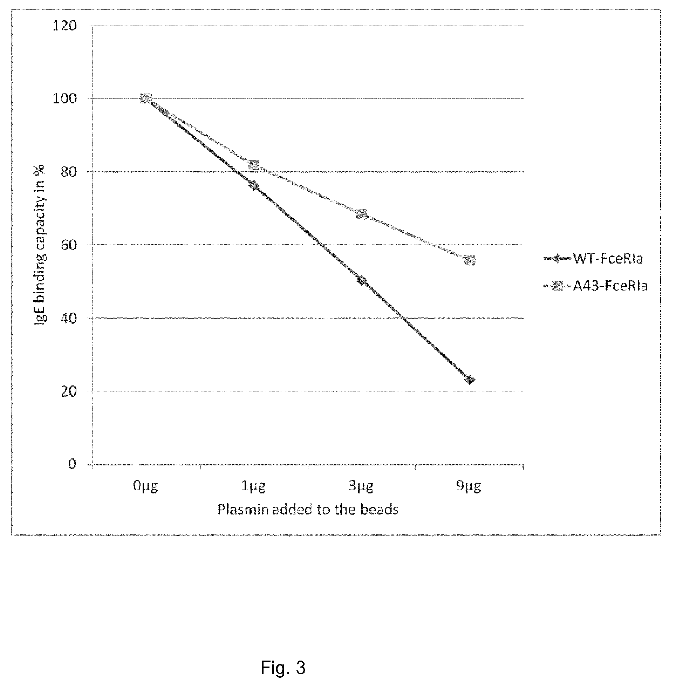

IgE binding capacity (expressed in %; see FIGURE 3) of FceRIa and protease resistant A43-FceRIa upon plasmin digestion of bead-immobilized adsorber protein. IgE binding capacity of FceRIa was markedly reduced after plasmin exposure when compared to protease resistant A43-FceRIa thereby showing increased sensitivity of the wt construct.

FIGURE 3 shows that IgE depletion is reduced to a different extent when incubating wild type or protease resistant A43-FceRIa adsorber with plasmin (dark and light line, respectively). After incubation with Plasmin at highest concentration, the IgE depletion capacity of the wt adsorber is reduced to 23% (corresponding to 12ng of the initial 50ng IgE), whereas the depletion capacity of the protease-resistant variant A43-FceRIa can be maintained at approximately 60%, respectively. The x-axis indicates the amount of plasmin (in µg) used for digesting the immobilized adsorber whereas the y-axis displays the relative IgE depletion capacity. In conclusion, plasmin digestion of wild type FceRIa adsorber reduces its IgE depletion capacity in a dose-dependent manner thereby stressing the importance to protect the adsorber molecule from proteolytic degradation and IgE binding capacity.

### Material and Methods:

Recombinant wild type FceRIa and A43-FceRIa protein was coupled to 1µm BcMag Iodoacetyl-activated magnetic beads (Bioclone FG-102) at 1-2mg Protein/ml beads according to the manufacturer's protocol. After blocking with 8mg/ml Cysteine, beads were washed 3x with PBS + 0.5% Tween (PBS-T). Same amount (18µg) of FceRIa coupled beads were incubated with 9µg/3µg/1µg and w/o Plasmin (Sigma P1867) in 1x Plasmin Digest Buffer ( 0.1M Tris-HCl, 2mM EDTA, pH 8) for 1h at RT/900rpm. After 3x washing with PBS-T, a 20% human serum solution in PBS-T spiked with 5µg/ml IgE was incubated with the coated beads for 1h/RT/900rpm. The IgE content of the IgE Mastermix was measured by ELISA before and after bead incubation, respectively, in order to determine IgE depletion efficiency of the adsorber. Relative depletion capacity was calculated from ELISA EC50 and the deduced IgE amount that remained in the supernatant *after* depletion with plasmin-treated adsorbers.

### IgE binding assay:

IgE binding was measured by ELISA while coating 50µl of a 2µg/ml BSW17 a mouse anti-human IgE antibody (NBS-C 0910-1-100) onto Maxisorp ELISA plates followed by blocking (with 1xPBS/1%BSA Blocking buffer) and incubation with "before and after bead incubation IgE samples" for 1 hour at RT. For IgE detection, 1µg/ml anti IgE-HRPO mAB (Novus Biologicals NBP1-74934) was subsequently incubated for 1 hour at RT followed by standard ABTS (Merck 8.22287.100) reaction (measured at OD405). EC50 were calculated on the basis of an nonlinear regression curve fitting model (Graphpad^{®}).

### EXAMPLE 4: Generation of a protease-resistant FceRIa variant.

In order to prevent cleavage of the prominent protease-sensitive K43 site by plasmin, it was necessary to exchange the positively charged cleavage site to a small, uncharged site at amino acid position 43 such as e.g. alanine, glycine, serine or tyrosine based on the knowledge about protein substrate specificity of plasmin as discussed above in EXAMPLE 2. In contrast, it is not desirable to exchange position K43 by a charged, aromatic, aliphatic amino acid or by cysteine since these amino acids might provide undesired protein folding artefacts (e.g. by unpredictable disulphide bonds) or since they might provide undesired neoepitopes because of their general propensity to participate in epitopes. It is known for example that proline or glycines frequently participates in core epitope structures as previously determined by Singh et al. 2013 possibly because of the formation of bends or flexibility in the epitope region. Such neoepitopes might become relevant to the organism upon shedding of the adsorber protein during apheresis treatment leading to induction of undesired antibodies against artificial neoepitopes present of the adsorber protein. In order to guarantee minimal structural changes for protein folding and antigenicity while at the same time minimizing the possibility of improper folding and the emergence of an undesired neoepitope, it is therefore necessary to mutagenize the K43 site most preferentially by alanine or alternatively by another small amino acid such as serine or tyrosine or possible but less preferably by glycine. As the most preferred paradigm, the K43→A43 mutation is presented in EXAMPLE 2 demonstrating that this substitution provides at the same time protease resistance while preserving full functionality for the IgE adsorption due to a minimal structural change of FceRIa. In contrast to many other mutations at different sites (see for example Mackay et al 2002), the most preferred K43→A43 modification of the present invention will not change the functional properties of FceRIa except that it renders the protein plasma protease resistant. Moreover this substitution will not influence precipitation propensity of recombinant FceRIa by mammalian expression systems as shown in EXAMPLE 2 and 4. The K43→A43 mutation provides protection from protease cleavage at K43 while maintaining IgE binding and proper refolding after denaturation/recycling as demonstrated in EXAMPLES 5-7. It is possible but not desirable and not necessary to substitute or delete the surrounding amino acids (i.e. up to 4 amino acid positions N-terminal or C-terminal of K43) in order to minimize the risk of structural changes and antigenicity at this site.

In order to prove practicability, an A43-FceRIa adsorber variant was constructed that cannot be cleaved by plasmin at position K43. The protease resistant adsorber provides increased stability, longer durability combined with a lowered risk of undesired shedding of adsorber fragments into the plasma during apheresis or a reduced propensity to degradation when applied as an administered biological therapeutic. At the same time the improved adsorber A43-FceRIa preserves its high affinity to IgE as shown in EXAMPLE 6.

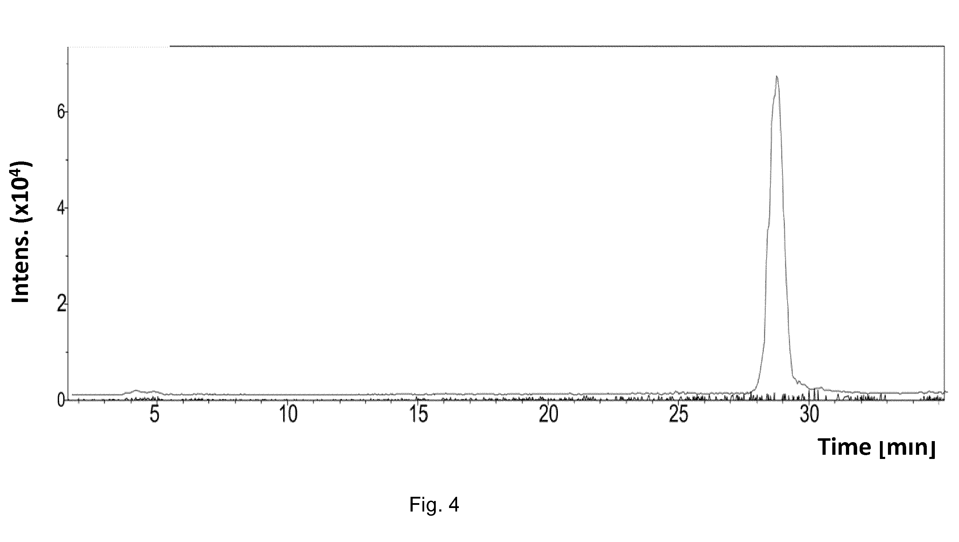

FIGURE 4 depicts a Base Peak Chromatogram showing the complete absence of a cleavage product in the mutant, protease resistant variant of recombinant A43-FceRIa (black line), whereas the wild type receptor digest peak appears at 30min (grey line).

### Material and Methods:

Analysis was performed by mass spectrometry following incubation of protease-resistant A43-FceRIa with plasmin as describe in EXAMPLE 2.

### EXAMPLE 5: A43-FceRIa does not change the properties of FceRIa and maintains its advantages over scFv12 (ELISA).

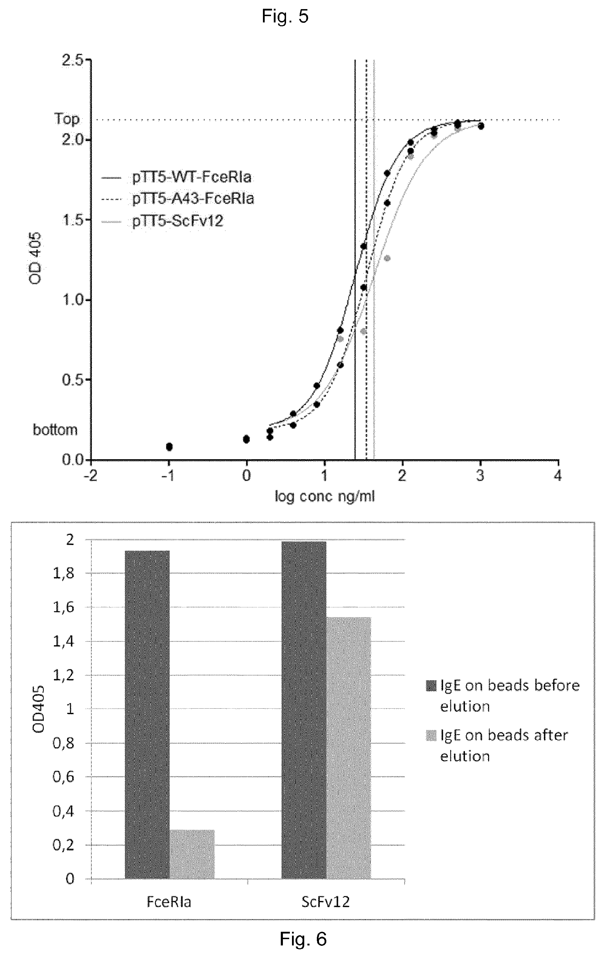

Protease resistant A43-FceRIa adsorber has similar affinity, avidity and specificity to IgE when compared to wild type FceRIa or prior art single chain antibody scFv12 (WO 2012/140214 A1) when comparing IgE binding by capture ELISA. Plates were coated with capturing antibody, after blocking HEK cell-purified recombinant protein derived from ScFv12 and FceRIa transfected cells, coated protein was incubated for 1 hour at RT. 1µg/ml IgE (NBS-C0911-0-100), trapped for 1h/RT and detected for IgE binding using 1µg/ml anti IgE-HRPO mAB (Novus Biologicals NBP1-74934) for 1 hour at RT. FIGURE 5 shows that calculated EC50 values (24.5ng/ml, 34,4ng/ml and 42,8ng/ml for FceRIa, A43-FceRIa and ScFv12, respectively) and curve shapes of IgE binding to A43-FceRIa are similar to wt-FceRIa or scFv12 demonstrating no negative effect of the K→A change at position 43 of the FceRIa sequence.

### EXAMPLE 6: Comparable affinities of A43-FceRIa, FceRIa and scFv12 to soluble IgE.

Affinity and kinetics of IgE/FceRIa and IgE/ScFv12 interactions. KD and association/dissociation kinetics of both recombinant FceRIa variants (i.e. wt and the A43 mutant) were highly comparable (KD∼4x10e-10), see EXAMPLE 6, TABLE 3 indicating that the AA-exchange at position 43 does not affect IgE binding capacity or quality. In TABLE 3 on- and off-rate values are listed together with calculated KDs for each of the 3 constructs. ScFv12 binding kinetics are different from FceRIa binding kinetics.

**Table 3**

| | **WT-FceRIa** | **A43-FceRIa** | **ScFv12** |
|---|---|---|---|
| **ka(1/MS)** | **1**,**10E+06** | **1,30E+06** | **4,11E+05** |
| **kd(1/s)** | **5,20E-04** | **4,92E-04** | **1,64E-04** |
| **KD** | **4,73E-10** | **3,79E-10** | **4,00E-10** |

For FceRIa variants, the on-rate was faster (ka-values ∼1x10e6) when compared to scFv12 (ka∼4x10e5). At the same time, the dissociation rate was faster in FceRIa variants (kd-values ∼5x10e-4) when compared to scFv12 (kd∼1,6x10e-4). On the other side, the off-rate of IgE binding was increased for FceRI when compared to scFv12. In principle, this could have implications for therapeutic apheresis settings where the faster IgE adsorption to the column is desired. Notably, a faster off-rate has significant practical implications for recycling of the adsorber (as demonstrated in EXAMPLE 7), for the flow rate in clinical apheresis and for the required coating density of the adsorber molecule on the solid support matrix of apheresis columns. Alternatively it could have significant impact when e.g. administering A43-FceRI as a biological therapeutic for IgE blocking.

### Material and Methods:

The surface of a Streptavidine (SA) sensor chip (GE Healthcare Biacore, Cat. Nr. BR-1000-32) was prepared by three injections with 20µl of 50mM NaOH following a 200µl injection of the biotinylated anti-Tag-mAB (Abcam ab10241) diluted to 2µg/ml in HBS-EP (0.01 M HEPES, 0.15 M NaCl, 3 mM EDTA, 0.005%(v/v) surfactant P20 (pH 7.4) (GE Healthcare Cat. Nr. BR-1001-88) resulting in an immobilization level of ∼2500 RU for capturing on flow cell 4. Free SA-binding sites were saturated by two consecutive 100µl injections of 1mM D-Biotin (Sigma #47868). Flow cell 3 was saturated with free D-Biotin and served as reference cell. The capturing level of FceRIa variants and of the ScFv12 required to obtain a maximal response of 100RU of IgE binding was calculated to be 75RU and 80RU, respectively. Therefore FceRIa, A43-FceRIa and scFv12 were diluted in HBS-EP and injected into flow cell 3 and 4 (30µl/min) until the immobilization level of 90RU was reached. After a stabilization time of 10 min with injection of running buffer, the final capturing level of 75 and 80RU respectively were obtained. Finally the individual interactions of FceRIa, A43-FceRIa and scFv12 with IgE (NBS-C0911-0-100) diluted in HBS-EP running buffer at 2-fold increasing concentrations (starting concentration, 0.5 nM; maximum concentration, 64 nM) were assessed with an IgE-association phase of 3min at a flow rate of 30µl/min. The dissociation phase was investigated by injection of HBS-EP for 30min at a flow rate of 30µl/min. The sensor chip surface was regenerated by injecting 15µl of 10 mM Glycine-HCl pH 1.8 at the same. The dissociation constant (KD-Value), on-rate (ka) and off-rate (kd) were calculated with BIAevaluation 4.1 (GE Healthcare Biacore) using a 1:1 interaction model. All measurements were performed on a Biacore 2000 device at 25°C.

### EXAMPLE 7: FceRIa is resistant to low pH treatment thereby allowing for recycling and quality monitoring of adsorption/desorption in clinical IgE apheresis.

Upon equal immobilization of FceRIa and scFv12 onto beads followed by *in vitro* IgE adsorption, IgE could be almost completely desorbed from FceRIa by pH 3.4 treatment whereas IgE was not released from ScFv12 as displayed in FIGURE 6. The y-axis of FIGURE 6 shows the IgE-signal measured on the beads before and after elution (dark and light bar, respectively). The x-axis indicates the immobilized adsorber used, respectively. This result is consistent with the shorter off-rate of FceRIa when compared to scFv12 as shown in EXAMPLE 6. Since low pH can destroy scFv12 (as shown in EXAMPLE 8), it is concluded that scFv12 is not suited for IgE desorption under low pH conditions consistent with the manufacturer's note that the scFv12-based apheresis column is designed for single use only (https://www.fresenius.com/5689 5968.htm). This unexpected advantage of FceRI-based adsorbers over the single chain-based adsorber represents an important practical aspect for recycling and reusing expensive apheresis columns. Moreover, exact quantification of desorbed IgE or autoantibody material after each apheresis cycle *without destroying the adsorber* might provide an extremely important clinical readout for monitoring treatment efficacy. According to the present invention, FceRI-based adsorbers, but not pH sensitive scFv-based adsorbers facilitate this aspect.

### Material and Methods:

Recombinant FceRIa and ScFv12 protein was coupled to 1µm BcMag Iodoacetyl-activated magnetic beads (Bioclone FG-102) at 1mg Protein/ml beads according to the manufacturer's protocol. Equal loading of proteins onto beads was assessed by a bead ELISA using 1µg/ml mouse anti FceRI antibody (Acris SM2251PS) and 50µl 0.4µg/ml goat a mouse IgG HRPO for detection. After blocking with 8mg/ml Cysteine, beads were washed 3x with PBS + 0.5% Tween. A 20% human serum solution in PBS-T spiked with 5µg/ml IgE was incubated with the coated beads for 1h/RT/900rpm. Beads were washed 3x with 300µl PBS-T and IgE was eluted with 100mM Glycine Buffer, pH 3.4 and neutralized with 0.5M Tris, pH 8. A bead ELISA was performed to check the IgE amount on the beads before and after elution. For the bead ELISA, the beads were washed 3x with PBS-T and blocked with blocking buffer (PBS-1%BSA) for 1h/RT/900rpm. 50µl of 1µg/ml IgE (NBS-C0911-0-100) was added to the beads and incubated 1h/RT/900rpm. Beads were washed 3x with PBS-0.5% Tween and incubated with 1µg/ml anti IgE-HRPO mAB (Novus Biologicals NBP1-74934) followed by standard ABTS (Merck 8.22287.100) reaction detection at 405nm.

### EXAMPLE 8: Low pH treatment of IgE adsorbers and regeneration with or without serum.

In order to monitor the adsorption efficacy in apheresis, it is not only necessary to measure plasma parameters but it is also necessary to provide quantitative information about the adsorbed material. It is preferable to provide an adsorber for clinical practice that does not need to be destroyed for analytical purposes. FceRIa fulfills these criteria and allows for desorption of IgE or possibly autoantibodies (as found e.g. in chronic autoimmune urticaria) for monitoring adsorption efficacy in clinical practice. Therefore it is necessary to provide an adsorber that should also not be too strong in order to apply practicable denaturing conditions between apheresis cycles. This can only be achieved if the adsorber has the capability to renature after desorption under harsh conditions such as e.g. low pH treatment without losing its binding capacity. At the same time the user of such an adsorber column must be sure at what extent the captured material has been released after recycling in order to allow quantification e.g. of the captured IgE or autoantibody material for clinical purpose and efficacy assessment. In order to test renaturation capabilities of wild type and protease resistant FceRIa adsorber against the prior art scFv12 adsorber, the IgE binding capacity of these adsorbers was directly compared after several cycles of denaturation/renaturation. Repeated injections of 100mM Glycine pH 1.8 moderately reduced IgE binding capacity of both FceRIa adsorbers whereas IgE binding capacity of ScFv12 was dramatically reduced to <15% as shown in FIGURES 7A. Furthermore, this experiment confirms that the modification providing protease resistance does not confer any disadvantage on pH sensitivity or IgE binding capacity to FceRIa-based adsorbers. FIGURE 7B shows the relative loss of IgE-binding under milder acidic conditions (pH 3) on a Biacore chip (without serum injection) corroborating the importance of denaturation/renaturation conditions under physiologic conditions for maintaining the capacity of the adsorber over several cycles. The stabilizing/renaturing effect of serum milieu for the protease resistant FceRI adsorber is reflected by moderate reduction in IgE-binding capacity in FIGURE 7A (with serum regeneration) compared to conditions without serum regeneration as depicted in FIGURE 7B. Consistently with the results from EXAMPLE 7, neither of these conditions could regenerate the prior art scFv12 adsorber after pH 3 or pH 1.8 desorption of IgE. Because of its improved stability A43-FceRIa is proposed as a recyclable alternative to wild type FceRIa or scFv12. The present invention provides improved cost of goods and facilitated monitoring of adsorption efficacy after each apheresis cycle. More generally, A43-FceRIa provides also improved blood-, plasma- or serum protease stability when intended as (component of a) biological therapeutic for injectable use.

### Material and Methods:

### Relative loss of IgE-binding capacity after repeated regenerations with pH 3 with or without serum injection using Biacore.

Thiol-ligand-coupling was performed according to the manufacturer's instructions (Thiol Coupling kit purchased from GE-Healthcare BR-1005-57). Briefly, the surface of the individual flow cell (Fc) of a CM5 sensor chip (GE Healthcare Biacore, BR1000-12) was activated by 35µl injection of a 1:1 mixture of 1-ethyl-3-(3-dimethylaminopropyl) carbodiimide hydrochloride (EDC) and N-hydroxysuccinimide (NHS) followed by a 35µl injection of 80mM 2-(2-Pyridinyldithio) ethaneamine hydrochloride (PDEA) dissolved in 50mM sodium borate buffer pH 8.5. Ligands were injected at a concentration of 10µg/ml dissolved in 50mM sodium acetate pH 4.5 to a level of∼1200RU. Chip surface was saturated by a 35µl injection of 50mM L-Cysteine resulting in a final immobilization level of ∼1000RU. Order of immobilized ligands: Fc1: K43del-FceRIa (i.e. a negative control, having a deletion from amino acid 1-37); Fc2: (wild type-) FceRIa; Fc3: (protease-resistant-) A43-FceRIa; Fc4: scFv12 (i.e. prior art monovalent IgE adsorber). The complete immobilization procedure was performed at a flow rate of 5µl/min using HBS-EP as running buffer. After immobilization, the stability of the IgE-binding capacity to ligands was assessed using repeated injections of 100mM Glycine pH 3 after IgE-binding. Therefore IgE (10µg/ml) was injected until saturation levels on Fc2, 3 and 4 were reached, following a 20µl injection of 100mM Glycine pH 3 (=cycle 1) as shown in FIGURE 7. Then again 600µl of IgE (10µg/ml) and 2x 5µl of 100mM Glycine pH 3 were injected (=cycle 2). The third IgE/regeneration cycle comprised 200µl IgE (10µg/ml) and 5µl of of 100mM Glycine pH 3 (=cycle 3) and the fourth IgE/regeneration cycle comprised 100µl IgE (10µg/ml) and 5µl of 100mM Glycine pH 3. Maximum IgE-binding capacity was assessed for each cycle. Reference subtracted RUs (Fc2-1, 3-1, 4-1) of maximum IgE-binding capacity for each ligand/flow cell were normalized to the saturation level before first regeneration with 100mM Glycine pH 3 on each flow cell and are indicated in percent in FIGURE 7A/7B. Flow rate and buffers used as in EXAMPLE 6.

### Relative loss of IgE-binding capacity after repeated regenerations with pH 3 with serum.

Chip immobilization was performed as described above for non-serum conditions. After immobilization 600µl of IgE (10µg/ml) followed by 50µl of pure human serum from healthy donors were injected and regeneration was performed using 10µl 100mM Glycine pH 1.8. Two more consecutive cycles of IgE/regeneration-injections with 600µl of IgE (10µl/mg) and 10µl of 100mM Glycine pH 1.8 respectively were performed and maximum IgE-binding capacity was assessed. Maximum IgE-binding capacity for each ligand/flow cell were normalized to the saturation level before first regeneration with 100mM Glycine pH 1.8 on each flow cell and are indicated in percent. All injections after immobilization were performed at a flow rate of 30µl/min using HBS-EP as running and dilution buffer.

### EXAMPLE 9: In vivo application of IgE apheresis using recombinant FceRIa.

In order to demonstrate the usefulness of recombinant FceRIa as selective IgE adsorber for extracorporal IgE depletion, 80µg human IgE (NBS-C0911-0-100) was injected via chronically implanted canules into the bloodstream of 6 Wistar rats. In this preclinical model for therapeutic apheresis, four animals were connected to the apheresis apparatus with an FceRIa- coupled CIM Monolith Column (BIA Separations #34003) and 2 control rats with Mock (empty) columns. Apheresis duration, flow rate and filtrated plasma volumes are indicated in TABLE 4.

**Table 4**

| Animal | filtrated Plasma volume (ml) | Apheresis Duration (min) | Flow rate (µl/min) | IgE in Eluate (µg) |
|---|---|---|---|---|
| FceRI-Rat-S 37 | 8,92 | 75 | 120 | 6,83 |
| FceRI-Rat-S 47 | 10,31 | 75 | 120-150 | 16,24 |
| FceRI-Rat-S 49 | 8,77 | 75 | 120 | 21,43 |
| FceRI-Rat-S 50 | 8,44 | 60 | 150 | 9,96 |

Using this *in vivo* setup (as previously described e.g. by Wallukat et al. 2012) between 6 and 21 µg of IgE (after subtracting the background signal of the "mock-rats" eluate from the apheresis columns) could be eluted, as indicated in the last column. In conclusion this demonstrates for the first time that FceRIa can be used for extracorporal IgE depletion from peripheral blood in an *in vivo* model for apheresis. Besides previous examples for the use as injectable therapeutic, this underpins the usefulness of FceRIa in therapeutic use.

### Material and methods:

Wistar rats with a body mass of ~250g were acclimatized for one week. Artery and vein catheters were implanted chronically one week before apheresis sessions started. The columns used were CIM Monolith Columns (BIA Separations 313.7175), which were coupled with FceRIa according to manufacturer's protocol. For blocking, 100mM Cysteine in 0.5M Na-Phosphate Buffer, pH 8.0 was used. Finally, 3.5mg protein was coupled to the 1ml column (according to the BIA Separations protocol). 2 animals constituted the control group that was treated with mock columns and 4 animals were used in the "treated group" (FceRIa columns) as indicated. Body weight was measured routinely 2-3x per week starting from implanting the canules and after apheresis IgE concentration in plasma was measured by ELISA as described in EXAMPLE 3. After observation, animals were narcotized (Sevorane) and via heart puncture, plasma was taken and frozen for further analysis. Apheresis setup: 5min before IgE application, blood serum was taken from the rats. At time point zero, 80µg human IgE (NBS-C0911-0-100) was applied for each animal. 15min/20min/25min after IgE application, blood samples were taken for plasma IgE determination. 30 minutes after IgE injection, animals were connected to the apheresis columns and apheresis took place for 60-75min depending on the flow rate. Blood samples were taken 30min after apheresis start during the procedure and at several time points after apheresis.

Preferred embodiments of the present invention are:
1. Alpha chain of the high-affinity IgE receptor (FceRIa), wherein the amino acid lysine at position 43 (K43) is exchanged with an amino acid selected from the group consisting of alanine, serine, tyrosine, isoleucine, leucine, asparagine, aspartic acid, methionine, phenylalanine, glutamic acid, threonine, glutamine, tryptophan, glycine, and valine, preferably alanine, glycine, serine or tyrosine, especially alanine.
2. FceRIa according to embodiment 1, having the amino acid sequence according to SEQ ID No. 1, wherein the amino acid lysine at position 43 (K43) is exchanged with an amino acid selected from the group consisting of alanine, serine, tyrosine, isoleucine, leucine, asparagine, aspartic acid, methionine, phenylalanine, glutamic acid, threonine, glutamine, tryptophan, glycine, and valine, preferably alanine, glycine, serine or tyrosine, especially alanine.
3. FceRIa according to embodiment 1 or 2, wherein a further amino acid is exchanged, preferably an amino acid selected from lysine at position 31 (K31), arginine at position 40 (R40), isoleucine at position 41 (I41), phenylalanine at position 42 (F42), glycine at position 44 (G44), glutamic acid at position 45 (E45), lysine at position 142 (K142), lysine at position 196 (K196), arginine at position 199 (R199), and lysine at position 201 (K201), especially selected from the group consisting of lysine at position 31 (K31), arginine at position 40 (R40), lysine at position 142 (K142), lysine at position 196 (K196), arginine at position 199 (R199), and lysine at position 201 (K201).
4. FceRIa according to any one of embodiments 1 to 3, wherein the FceRIa is immobilised on a solid surface.
5. FceRI comprising an alpha chain, a beta chain and two gamma chains connected by two disulfide bridges, wherein the alpha chain is a modified alpha chain according to any one of embodiments 1 to 4.
6. FceRIa according to any one of embodiments 1 to 4, for use in the prevention and/or treatment of IgE mediated diseases, wherein the FceRIa is used for depletion of excess IgE (anti IgE therapy) from human body fluids, especially human plasma or serum, in apheresis.
7. FceRIa for use according to embodiment 6, wherein the IgE mediated disease is selected from the group consisting of allergic diseases, preferably seasonal, food, pollen, mold spores, poison plants, medication/drug, insect-,scorpion- or spider-venom, latex or house dust mite allergies, pet allergies, allergic rhinitis and -conjunctivitis, allergic conjunctivitis, allergic asthma bronchiale, non-allergic asthma, Churg-Strauss Syndrome, atopic dermatitis, nasal polyposis, Kimura's disease, contact dermatitis to adhesives, antimicrobials, fragrances, hair dye, metals, rubber components, topical medicaments, rosins, waxes, polishes, cement and leather, chronic rhinosinusitis, atopic eczema, IgE related autoimmune diseases, preferably chronic (idiopathic) and autoimmune urticaria, cholinergic urticaria, mastocytosis, especially cutaneous mastocytosis, allergic bronchopulmonary aspergillosis, chronic or recurrent idiopathic angioedema, interstitial cystitis, anaphylaxis, especially idiopathic and exercise-induced anaphylaxis, immunotherapy, eosinophil-associated diseases, preferably eosinophilic asthma, eosinophilic gastroenteritis, eosinophilic otitis media and eosinophilic oesophagitis; lymphomas, sensibilisation side effects of an anti-acidic treatment, preferably for gastric or duodenal ulcer or reflux.
8. FceRIa for use according to embodiment 6 or 7, wherein the FceRIa is coupled to a solid carrier which is suitable for contacting with the blood stream of a human individual.
9. An apheresis device comprising a solid carrier capable of being contacted with the blood or plasma flow, characterised in that the solid carrier includes an FceRIa according to any one of embodiments 1 to 4.
10. The device according to embodiment 9, characterised in that the carrier is a sterile and pyrogen-free column.
11. The device according to embodiment 9 or 10, characterised in that it comprises further IgE binding molecules.
12. Use of a device according to any one of embodiments 9 to 11 for providing a prevention and/or treatment device for preventing and/or treating an IgE related disease, especially for performing an anti IgE therapy.
13. A kit for use in preventing and/or treating IgE related diseases comprising a solid apheresis carrier containing FceRIa according to any one of embodiments 1 to 4, wherein said carrier is a sterile and pyrogen-free column.
14. FceRIa according to any one of embodiments 1 to 4 for use in a therapeutic method, especially for use in the prevention or treatment of IgE related diseases.
15. FceRIa for use according to embodiment 14 as an injectable biological therapeutic.
16. FceRIa for use according to embodiment 14 or 15, used in combination with a further IgE lowering therapy.
17. Pharmaceutical composition comprising FceRIa according to any one of embodiments 1 to 4 and a pharmaceutically acceptable carrier.
18. Pharmaceutical composition according to embodiment 17, further comprising an agent which increases the half-life in a patient to whom the composition is administered.
19. Pharmaceutical composition according to embodiment 18, wherein the agent which increases the half-life is human serum albumin (HSA) or a chemical modification such as PEGylation.
20. Use of the FceRIa according to any one of embodiments 1 to 4 as a recyclable probe for IgE detection in protease containing samples, especially plasma samples.
21. Use according to embodiment 20, wherein the probe is used in an ELISA or SPR assay.
22. FceRIa according to any one of embodiments 1 to 4, coupled to
   - a linker molecule, preferably a peptide linker, especially a peptide linker consisting of one to ten, preferably two to five, amino acid residues; and/or
   - a therapeutic or diagnostic molecule, preferably a monoclonal antibody or antibody fragment, a cytokine, an antibody-like structure, a cytotoxic or inhibitory agent, an optical tracer; and/or
   - a carrier, preferably a carrier protein, especially human serum albumin, transferrin or a Fc domain.
23. FceRIa according to embodiment 22, wherein the FceRIa is coupled to a cytotoxic agent.
24. FceRIa according to embodiment 22 or 23, for use in the treatment of allergy or other IgE mediated diseases, wherein FceRIa is used for delivering cytotoxic or inhibitory agents to IgE B-cell receptor expressing cells in order to prevent differentiation of these cells to IgE producing plasma cells.
25. FceRIa according to any one of embodiments 22 to 24, for use in the treatment of a disease selected from the group consisting of allergic diseases, preferably seasonal, food, pollen, mold spores, poison plants, medication/drug, insect-,scorpion- or spider-venom, latex or house dust mite allergies, pet allergies, allergic rhinitis and -conjunctivitis, allergic conjunctivitis, allergic asthma bronchiale, non-allergic asthma, Churg-Strauss Syndrome, atopic dermatitis, nasal polyposis, Kimura's disease, contact dermatitis to adhesives, antimicrobials, fragrances, hair dye, metals, rubber components, topical medicaments, rosins, waxes, polishes, cement and leather, chronic rhinosinusitis, atopic eczema, IgE related autoimmune diseases, preferably chronic (idiopathic) and autoimmune urticaria, cholinergic urticaria, mastocytosis, especially cutaneous mastocytosis, allergic bronchopulmonary aspergillosis, chronic or recurrent idiopathic angioedema, interstitial cystitis, anaphylaxis, especially idiopathic and exercise-induced anaphylaxis, immunotherapy, eosinophil-associated diseases, preferably eosinophilic asthma, eosinophilic gastroenteritis, eosinophilic otitis media and eosinophilic oesophagitis; lymphomas, sensibilisation side effects of an anti-acidic treatment, preferably for gastric or duodenal ulcer or reflux.

### REFERENCES:

Backes et al.; Synthesis of positional-scanning libraries of fluorogenic peptide substrates to define the extended substrate specificity of plasmin and thrombin. Nat Biotechnol. 2000 Feb;18(2):187-93. Erratum in: Nat Biotechnol 2000 May;18(5):559. PubMed PMID:10657126
Bennich & Ishizaka et al.; Immunoglobulin E. A new class of human immunoglobulin. Immunochemistry. 1968 Jul;5(4):327-8. PubMed PMID: 4103909
Bootz F, Neri D. Immunocytokines: a novel class of products for the treatment of chronic inflammation and autoimmune conditions. Drug Discov Today. 2015 Oct 23. Review. PMID: 26526566
Botelho et al.; Top-down and bottom-up proteomics of SDS-containing solutions following mass-based separation. J Proteome Res. 2010 Jun 4;9(6):2863-70. doi: 10.1021/pr900949p. PubMed PMID: 20377267.
Chen et al. Fusion protein linkers: property, design and functionality. Adv Drug Deliv Rev. 2013 Oct;65(10):1357-69. Review. PMID: 23026637
Derfler et al.; Effective and safe in vivo IgE-depletion by a novel IgE-adsorber (IgEnio), EAACI Online Library, Jun 6, 2015; 103776
Digan et al.; Patent WO 1998/004718 A1
Dullaers et al.; The who, where, and when of IgE in allergic airway disease. J Allergy Clin Immunol.2012, 129(3):635-45. PubMed PMID: 22168998.
Galli & Tsai; IgE and mast cells in allergic disease. Nat Med. 2012;18(5):693-704. doi: 10.1038/nm.2755. Review. PubMed PMID: 22561833
Garman et al.; Crystal structure of the human high-affinity IgE receptor. Cell. 1998 Dec 23;95(7):951-61. PubMed PMID: 9875849.
Gould et al.; Patent WO 99/05271 A1
Hervio LS, et al.; Negative selectivity and the evolution of protease cascades: the specificity of plasmin for peptide and protein substrates. Chem Biol. 2000 7(6):443-53. PubMed PMID: 10873836.
Hogarth et al.; Patent WO 96/08512 A1, US 8,729,247 B2 (Austin Research Institute)
Holgate; World Allergy Organ J. 2014 Jul 29;7(1):17. doi: 10.1186/1939-4551-7-17. eCollection 2014. Review. PMID: 25097719
Huber et al.; Patent WO 2000/032767 A1
Incorvaia et al.; Omalizumab, an anti immunoglobulin E antibody: state of the art. Drug Des Devel Ther. 2014 Feb 7;8:197-207. doi: 10.2147/DDDT.S49409. eCollection 2014. PubMed PMID: 24532966; PubMed Central PMCID: PMC3923619.
Kasperkiewicz et al.; Improvement of treatment-refractory atopic dermatitis by immunoadsorption: a pilot study. J Allergy Clin Immunol. 2011 Jan;127(1):267-70, 270.e1-6. doi: 10.1016/j.jaci.2010.07.042. Epub 2010 Oct 20. PubMed PMID: 20970174.
Kerzel et al.; Plasmapheresis prior to omalizumab administration in a 15-year-old boy with severe asthma and very high IgE levels: sustained effect over 2 years. Klein Padiatr. 2011 Nov;223(6):356-9. doi: 10.1055/s-0031-1287824. Epub 2011 Oct 19., PubMed PMID: 22012605.
Lebedin et al.; Ex vivo removal of IgE in atopic asthma by extracorporeal plasmoimmunoadsorption (EPIA): development of a clinical adsorbent. Int J Artif Organs. 1991 Aug;14(8):508-14. PubMed PMID:1937940.
Licari et al.; The discoveryand development of omalizumab for the treatment of asthma. Expert Opin Drug Discov. 2015;10(9):1033-42. doi: 10.1517/17460441.2015.1048220. Epub 2015 May 15. PubMed PMID: 25979110.
Lingyun Jia et al.; Patent CN 102660569 A
Lowe et al.; Revision of omalizumab dosing table for dosing every 4 instead of 2 weeks for specific ranges of bodyweight and baseline IgE. Regul Toxicol Pharmacol. 2015 Feb;71(1):68-77. doi:10.1016/j.yrtph.2014.12.002. Epub 2014 Dec 8. PubMed PMID: 25497995.
Lupinek et al.; Trimolecular complex formation of IgE, Fc epsilon RI, and arecombinant nonanaphylactic single-chain antibody fragment with high affinity forIgE. J Immunol. 2009 Apr 15;182(8):4817-29. doi: 10.4049/jimmunol.0800726. PubMedPMID: 19342660.
Lupinek et al.; WO 2012/140214 A1; Patent US 2014/0124448 A1
Mackay et al.; Mutagenesis within human FcepsilonRIalpha differentially affects human and murine IgE binding. J Immunol. 2002 Feb 15;168(4):1787-95. PubMed PMID: 11823511.
Mallamaci et al.; Identification of sites on the human Fc epsilon RI alpha subunit which are involved in binding human and rat IgE. J Biol Chem. 1993 Oct 15;268(29):22076-83. PubMed PMID: 8408065.
McKenzie et al.; Patent US 5,985,599 A
Miller et al.; Expression of high-affinity binding of human immunoglobulin E by transfected cells. Science. 1989 Apr 21;244(4902):334-7. PubMed PMID: 2523561.
Oliveira S; Considerations on the Advantages of Small Tracers for Optical Molecular Imaging. J Mol Biol & Mol Imaging. 2015;2(2): 1016.
Pearson et al.; J. Immunoglobulin E in irritable bowel syndrome: another target for treatment? A case report and literature review. Therap Adv Gastroenterol. 2015 Sep;8(5):270-7. doi: 10.1177/1756283X15588875. Review. PubMed PMID: 26327917; PubMed Central PMCID:PMC4530434.
Perkins et al.; Probability-based protein identification by searching sequence databases using mass spectrometry data. Electrophoresis. 1999 Dec;20(18):3551-67. PubMed PMID: 10612281.
Peters C and Brown S. Antibody-drug conjugates as novel anticancer chemotherapeutics. Biosci Rep. 2015 Jun 12;35(4). Review. PMID: 26182432
Platzer et al., Soluble IgE receptors-elements of the IgE network. Immunology Letters [2011, 141(1):36-44] 2012/01, PubMed PMID:21920387
Robertson; Phage and Escherichia coli expression of the human high affinity immunoglobulin E receptor alpha-subunit ectodomain. Domain localization of the IgE-binding site. J Biol Chem. 1993 Jun 15;268(17):12736-43. PubMed PMID:8509408.
Sato et al.; Specific removal of IgE by therapeutic immunoadsorption system. J Immunol Methods. 1989 Mar 31;118(2):161-8. PubMed PMID: 2647856.
Shroba et al.; J. Current treatment options for idiopathic angioedema. Ann Allergy Asthma Immunol. 2015 Sep 1. pii: S1081-1206(15)00522-0. doi: 10.1016/j.anai.2015.07.023. [Epub ahead of print] PubMed PMID: 26341649.
Singh et al.; Improved method for linear B-cell epitope prediction using antigen's primary sequence. PLoS One. 2013 May 7;8(5):e62216. doi: 10.1371/journal.pone.0062216. Print 2013. PubMed PMID: 23667458; PubMed, Central PMCID: PMC3646881.
Siraganian et al.; Patent WO 89/05352 A1
Spiess C et al. Alternative molecular formats and therapeutic applications for bispecific antibodies. Mol Immunol. 2015 Oct;67(2 Pt A):95-106. Review. PMID: 25637431
Sutton et al.; Structure and dynamics of IgE-receptor interactions: FcεRI and CD23/FcεRII. Immunol Rev. 2015 Nov;268(1):222-35. doi: 10.1111/imr.12340. Review. PubMed PMID: 26497523.
Van Vught R et al.; Site-specific functionalization of proteins and their applications to therapeutic antibodies. Comput StructBiotechnol J. 2014 Feb 14;9:e201402001. doi: 10.5936/csbj.201402001. eCollection 2014. Review. PubMed PMID: 24757499; PubMed Central PMCID: PMC3995230.
Wallukat et al.; The first aptamer-apheresis column specifically for clearing blood of β1-receptor autoantibodies. Circ J. 2012;76(10):2449-55. Epub 2012 Jul 27. PubMed PMID: 22850243.
Wurzburg et al.; Structural insights into the interactions between human IgE and its high affinity receptor FcepsilonRI. Mol Immunol. 2002 May;38(14):1063-72. Review. PubMed PMID: 11955598.
Yanagihara et al.; Recombinant soluble form of the human high-affinity immunoglobulin E (IgE) receptor inhibits IgE production through its specific binding to IgE-bearing B cells. J Clin Invest. 1994 Nov;94(5):2162-5. PubMed PMID: 7525655; PubMed Central PMCID:PMC294671.
Yuan et al.; The serine protease plasmin cleaves the amino-terminal domain of the NR2A subunit to relieve zinc inhibition of the N-methyl-D-aspartate receptors. J Biol Chem. 2009 May 8; 284(19):12862-73. doi: 10.1074/jbc.M805123200. Epub 2009 Feb 24. PubMed PMID: 19240037
Zink et al.; Targeting IgE in Severe Atopic Dermatitis with a Combination of Immunoadsorption and Omalizumab. Acta Derm Venereol. 2015 Jun 10. doi: 10.2340/00015555-2165. [Epub ahead of print] PubMed PMID: 26059424
Zink Alexander; Pilotstudie zur experimentellen Kombinationstherapie von Ig-Apherese und Omalizumab bei schwerem Atopischem Ekzem mit erhöhten IgE-Spiegeln, 2012, PhD Thesis https://mediatum.ub.tum.de/doc/ 1085022/ 1085022.pdf
Zuberbier T, Henz BM, Fiebiger E, Maurer D, Stingl G. Anti-FcepsilonRIalpha serum autoantibodies in different subtypes of urticaria. Allergy. 2000 Oct; 55(10):951-4. PubMed PMID: 11030376.

## Claims

1. Alpha chain of the high-affinity IgE receptor (FceRIa), wherein the amino acid lysine at position 43 (K43) is exchanged with an amino acid selected from the group consisting of alanine, serine, tyrosine, isoleucine, leucine, asparagine, aspartic acid, methionine, phenylalanine, glutamic acid, threonine, glutamine, tryptophan, glycine, and valine, preferably alanine, glycine, serine or tyrosine, especially alanine.

2. FceRIa according to claim 1, having the amino acid sequence according to SEQ ID No. 1, wherein the amino acid lysine at position 43 (K43) is exchanged with an amino acid selected from the group consisting of alanine, serine, tyrosine, isoleucine, leucine, asparagine, aspartic acid, methionine, phenylalanine, glutamic acid, threonine, glutamine, tryptophan, glycine, and valine, preferably alanine, glycine, serine or tyrosine, especially alanine.

3. FceRIa according to claim 1 or 2, wherein a further amino acid is exchanged, preferably an amino acid selected from lysine at position 31 (K31), arginine at position 40 (R40), isoleucine at position 41 (I41), phenylalanine at position 42 (F42), glycine at position 44 (G44), glutamic acid at position 45 (E45), lysine at position 142 (K142), lysine at position 196 (K196), arginine at position 199 (R199), and lysine at position 201 (K201), especially selected from the group consisting of lysine at position 31 (K31), arginine at position 40 (R40), lysine at position 142 (K142), lysine at position 196 (K196), arginine at position 199 (R199), and lysine at position 201 (K201).

4. FceRIa according to any one of claims 1 to 3, wherein the FceRIa is immobilised on a solid surface.

5. FceRI comprising an alpha chain, a beta chain and two gamma chains connected by two disulfide bridges, wherein the alpha chain is a modified alpha chain according to any one of claims 1 to 4.

6. FceRIa according to any one of claims 1 to 4, for use in the prevention and/or treatment of IgE mediated diseases, wherein the FceRIa is used for depletion of excess IgE (anti IgE therapy) from human body fluids, especially human plasma or serum, in apheresis.

7. FceRIa for use according to claim 6, wherein the IgE mediated disease is selected from the group consisting of allergic diseases, preferably seasonal, food, pollen, mold spores, poison plants, medication/drug, insect-,scorpion- or spider-venom, latex or house dust mite allergies, pet allergies, allergic rhinitis and -conjunctivitis, allergic conjunctivitis, allergic asthma bronchiale, non-allergic asthma, Churg-Strauss Syndrome, atopic dermatitis, nasal polyposis, Kimura's disease, contact dermatitis to adhesives, antimicrobials, fragrances, hair dye, metals, rubber components, topical medicaments, rosins, waxes, polishes, cement and leather, chronic rhinosinusitis, atopic eczema, IgE related autoimmune diseases, preferably chronic (idiopathic) and autoimmune urticaria, cholinergic urticaria, mastocytosis, especially cutaneous mastocytosis, allergic bronchopulmonary aspergillosis, chronic or recurrent idiopathic angioedema, interstitial cystitis, anaphylaxis, especially idiopathic and exercise-induced anaphylaxis, immunotherapy, eosinophil-associated diseases, preferably eosinophilic asthma, eosinophilic gastroenteritis, eosinophilic otitis media and eosinophilic oesophagitis; lymphomas, sensibilisation side effects of an anti-acidic treatment, preferably for gastric or duodenal ulcer or reflux.

8. FceRIa for use according to claim 6 or 7, wherein the FceRIa is coupled to a solid carrier which is suitable for contacting with the blood stream of a human individual.

9. An apheresis device comprising a solid carrier capable of being contacted with the blood or plasma flow, **characterised in that** the solid carrier includes an FceRIa according to any one of claims 1 to 4.

10. Use of a device according to claim 9 for providing a prevention and/or treatment device for preventing and/or treating an IgE related disease, especially for performing an anti IgE therapy.

11. A kit for use in preventing and/or treating IgE related diseases comprising a solid apheresis carrier containing FceRIa according to any one of claims 1 to 4, wherein said carrier is a sterile and pyrogen-free column.

12. FceRIa according to any one of claims 1 to 4 for use in a therapeutic method, especially for use in the prevention or treatment of IgE related diseases.

13. Pharmaceutical composition comprising FceRIa according to any one of claims 1 to 4 and a pharmaceutically acceptable carrier.

14. FceRIa according to any one of claims 1 to 4, coupled to
- a linker molecule, preferably a peptide linker, especially a peptide linker consisting of one to ten, preferably two to five, amino acid residues; and/or
- a therapeutic or diagnostic molecule, preferably a monoclonal antibody or antibody fragment, a cytokine, an antibody-like structure, a cytotoxic or inhibitory agent, an optical tracer; and/or
- a carrier, preferably a carrier protein, especially human serum albumin, transferrin or a Fc domain.

15. FceRIa according to claim 14, for use in the treatment of a disease selected from the group consisting of allergic diseases, preferably seasonal, food, pollen, mold spores, poison plants, medication/drug, insect-,scorpion- or spider-venom, latex or house dust mite allergies, pet allergies, allergic rhinitis and -conjunctivitis, allergic conjunctivitis, allergic asthma bronchiale, non-allergic asthma, Churg-Strauss Syndrome, atopic dermatitis, nasal polyposis, Kimura's disease, contact dermatitis to adhesives, antimicrobials, fragrances, hair dye, metals, rubber components, topical medicaments, rosins, waxes, polishes, cement and leather, chronic rhinosinusitis, atopic eczema, IgE related autoimmune diseases, preferably chronic (idiopathic) and autoimmune urticaria, cholinergic urticaria, mastocytosis, especially cutaneous mastocytosis, allergic bronchopulmonary aspergillosis, chronic or recurrent idiopathic angioedema, interstitial cystitis, anaphylaxis, especially idiopathic and exercise-induced anaphylaxis, immunotherapy, eosinophil-associated diseases, preferably eosinophilic asthma, eosinophilic gastroenteritis, eosinophilic otitis media and eosinophilic oesophagitis; lymphomas, sensibilisation side effects of an anti-acidic treatment, preferably for gastric or duodenal ulcer or reflux.
